# EUROPEAN PATENT APPLICATION

(11) **EP 1 783 216 A1**
(43) Date of publication of application: **09.05.2007**
(21) Application number: 05743855.8
(22) Date of filing: 26.05.2005
(51) Int. Cl.: C12N 15/09, A61K 31/7125, A61K 48/00, A61P 35/00, A61P 43/00, C07H 21/00

(54) **TELOMERASE-INHIBITORY ENA OLIGONUCLEOTIDE**

(30) Priority: 28.05.2004 JP 2004159784
(71) Applicant: Sankyo Company, Limited, Tokyo 103-8426 (JP)
(72) Inventor: KOIZUMI, Makoto, Shinagawa-ku, Tokyo 140-8410 (JP); ANDO, Osamu, Shinagawa-ku, Tokyo 140-8410 (JP)
(74) Representative: Gibson, Christian John Robert
(86) International application number: PCT/JP2005/009664
(87) International publication number: WO 2005/116207

(57) **Abstract**

A compound represented by general formula:

E1-B1-B2-B3-B4-E2 (I)

(wherein E1 represents a group represented by the formula R1- or the like; E2 represents a group represented by the formula -B7-R2 or the like; B4, B5, and B8 are identical or different, and each represents T^{p} or the like; B1, B2, B3, and B12 are identical or different, and each represents G^{p} or the like; B 16 represents C^{p} or the like; and B6, B10, B14, and B18 are identical or different, and each represents A^{p} or the like) or a pharmacologically acceptable salt thereof, inhibits the action of telomerase and is effective in treating diseases in which telomerase is involved.

## Description

### Technical Field

The present invention relates to a telomerase-inhibiting ENA oligonucleotide compound or a pharmacologically acceptable salt thereof inhibiting the action of telomerase specifically expressed in cancer cells and effective in treating and preventing diseases such as cancer, to a medicinal composition containing the same as an active ingredient, to the use of the compound or a pharmacologically acceptable salt thereof for producing the medicinal composition, and to a method for preventing or treating diseases which involves administering a pharmacologically effective amount of the compound or a pharmacologically acceptable salt thereof to a warm-blooded animal (particularly, human).

### Background Art

The end of a chromosome is called the "telomere". In cell division, the telomere is shortened owing to the incomplete replication of chromosomal DNA during mitosis. When the telomere reaches a certain critical length, the remaining DNA becomes unstable and cells containing the shortened DNA typically enter into senescence and end up in death. Such cell senescence or death is not observed in cells containing telomerase. Telomerase is a ribonucleoprotein enzyme using a sequence contained in the RNA component of the enzyme as a template to synthesize one strand of telomeric DNA. It is probable that cells having telomerase activities typically do not undergo senescence and therefore can maintain immortality. Telomerase activities have been found in more than 85% of all malignant tumors including skin, connective tissue, depot fat, breast, lung, stomach, pancreas, ovary, cervical, uterine, kidney, bladder, colon, prostate, central nervous system, retina, and blood tumor cell line, and, in most cases, have not been observed in normal cells (see Feng, et al., Science, 269: 1236-1241, 1995; Kim et al., Science, 266: 2011-2014, 1994; and PCT application WO93/23572 published November 25, 1993).

Thus, a compound inhibiting telomerase is considered to effectively treat or prevent cancer and the like.

As a compound inhibiting telomerase, there is known a compound having the sequence: 5'-tagggttagacaa-3' (SEQ ID NO: 1 of the Sequence Listing) (hereinafter referred to as "compound A") that complementarily binds to a certain region of the template RNA in the telomerase, whose nucleosides are bound to each other through thio-phosphoramidate bonds (Asai et al., Cancer Research, 63: 3931-3939, 2003).
Non patent literature 1: Science, 269, 1236-1241 (1995)
Non patent literature 2: Science, 266, 2011-2014 (1994)
Patent literature 1: WO 93/23572
Non patent literature 3: Cancer Research, 63, 3931-3939 (2003)

### Disclosure of the Invention

Compound A, previously known, has not been sufficient in activity and stability. Thus, a compound having improved activity and stability has been hoped for.

As the result of studying substances inhibiting the action of telomerase, the present inventors have found that a compound of general formula (I) strongly binds to telomerase RNA and that it is useful for treating diseases in which telomerase is involved, such as cancer, thereby accomplishing the invention.

Thus, the present invention is a compound having general formula (I) below:

E1-B1-B2-B3-B4-E2 (I)

(wherein E1 represents a group represented by the formula R1-, a group represented by the formula R1-B6-, or a group represented by the formula R1-B5-B6-;

E2 represents a group represented by the formula -B7-R2, a group represented by the formula -B8-B9-R2, a group represented by the formula -B8-B10-B11-R2, a group represented by the formula -B8-B10-B12-B13-R2, a group represented by the formula -B8-B10-B12-B14-B15-R2, a group represented by the formula -B8-B10-B12-B14-B16-B17-R2, or a group represented by the formula - B8-B10-B12-B14-B16-B18-B19-R2;

B4, B5, and B8 are identical or different, and each represents a group represented by formula: 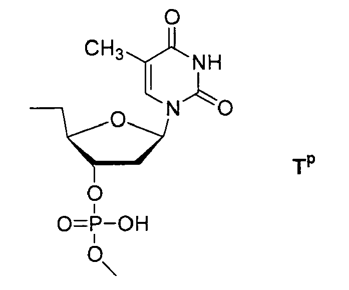 (hereinafter referred to as "T^{p}"), a group represented by formula: 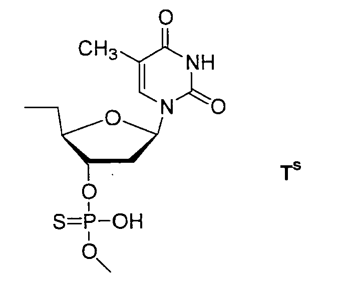 (hereinafter referred to as "T^{s}"), a group represented by general formula T^{ep}: 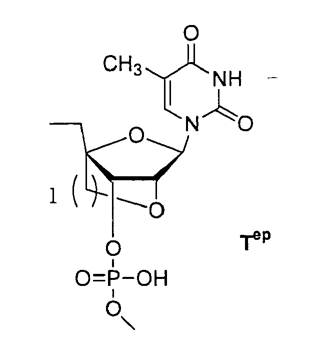 (where 1 represents an integer of 1 to 5), or a group represented by general formula T^{es}: 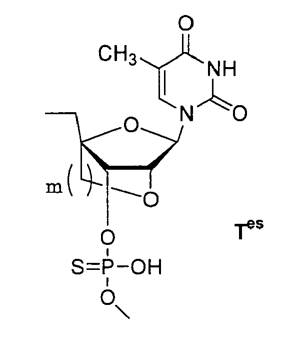 (where m represents an integer of 1 to 5);

B1, B2, B3, and B12 are identical or different, and each represents a group of formula: 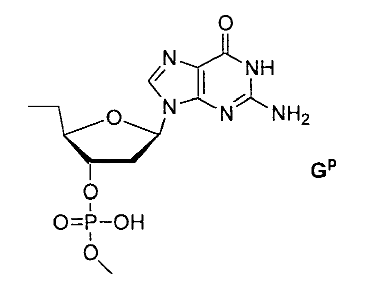 (hereinafter referred to as "G^{p}"), a group represented by formula: 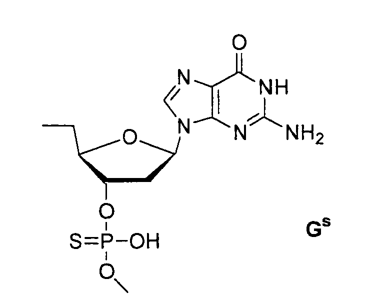 (hereinafter referred to as "G^{s}"), a group represented by general formula G^{ep}: 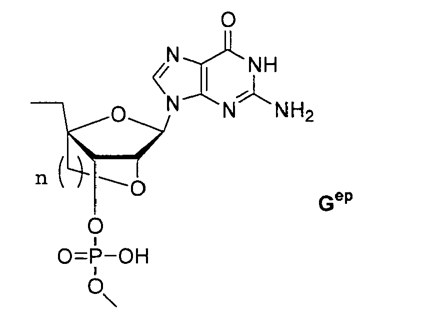 (where n represents an integer of 1 to 5), or a group represented by general formula G^{es}: 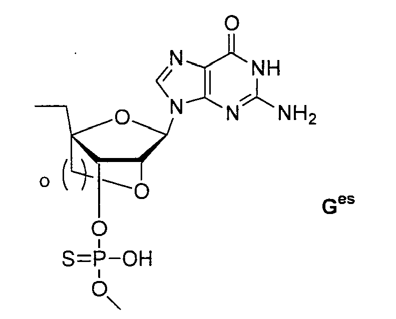 (where o represents an integer of 1 to 5);

B 16 represents a group represented by formula: 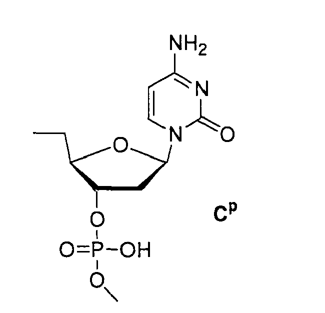 (hereinafter referred to as "C^{p}"), a group represented by formula: 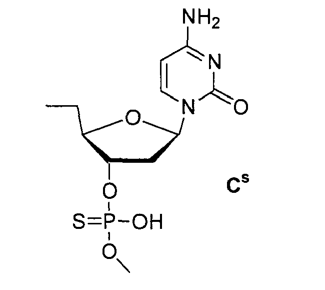 (hereinafter referred to as "C^{s}"), a group represented by general formula C^{ep}: 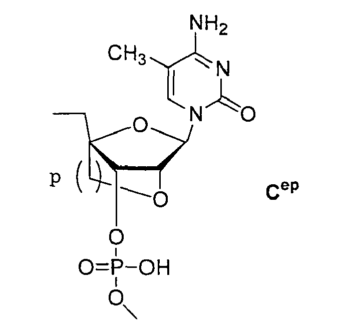 (where p represents an integer of 1 to 5), or a group represented by general formula C^{es}: 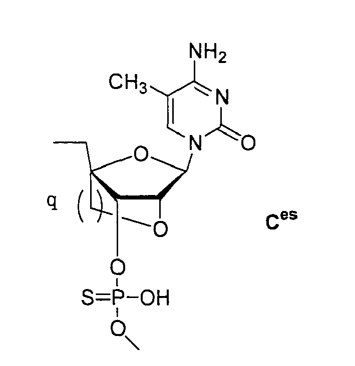 (where q represents an integer of 1 to 5);

B6, B10, B 14, and B 18 are identical or different, and each represents a group represented by formula: 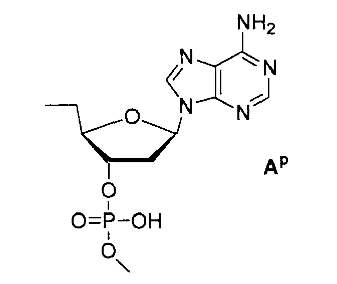 (hereinafter referred to as "A^{p}"), a group represented by formula: 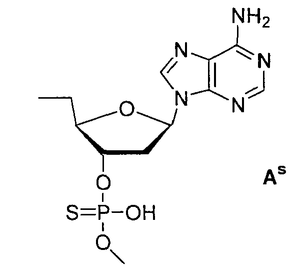 (hereinafter referred to as "A^{s}"), a group represented by general formula A^{ep}: 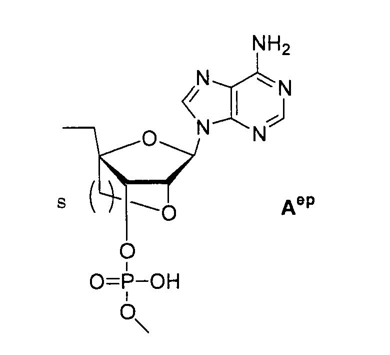 (where s represents an integer of 1 to 5), or a group represented by general formula A^{es}: 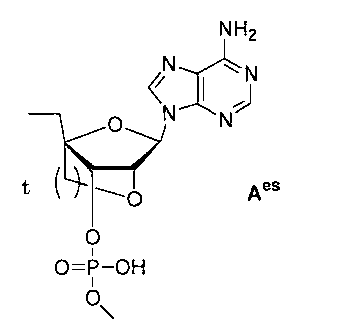 (where t represents an integer of 1 to 5);

B 11 represents a group represented by formula: 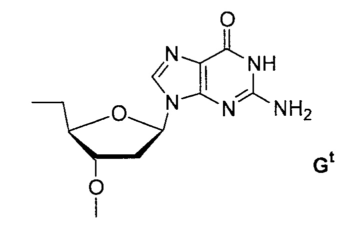 (hereinafter referred to as "G^{t}") or a group represented by general formula G^{et}: 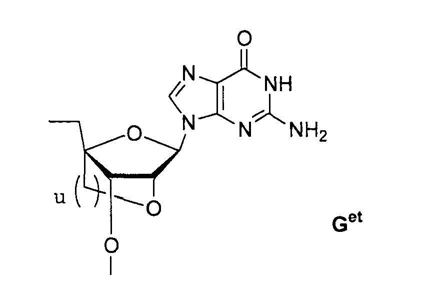 (where u represents an integer of 1 to 5);

B 15 represents a group represented by formula: 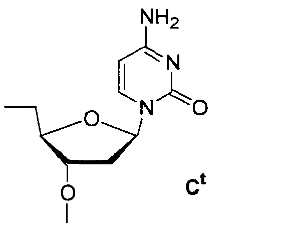 (hereinafter referred to as "C^{t}") or a group represented by general formula C^{et}: 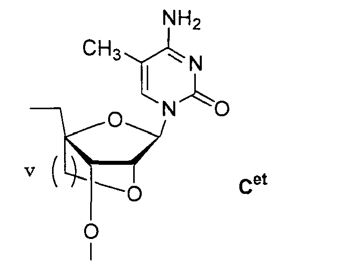 (where v represents an integer of 1 to 5);

B9, B 13, B 17, and B 19 are identical or different, and each represents a group represented by formula: 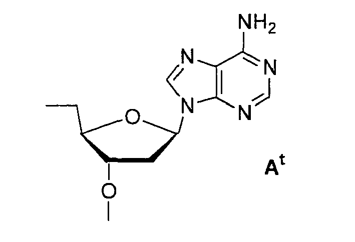 (hereinafter referred to as "A^{t}") or a group represented by general formula A^{et}: 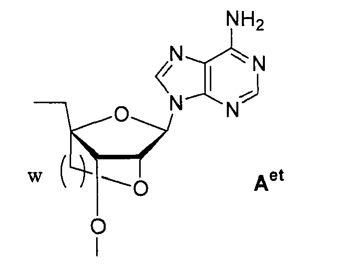 (where w represents an integer of 1 to 5);

B7 represents a group represented by formula: 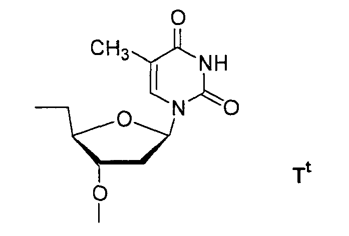 (hereinafter referred to as "T^{t}") or a group represented by general formula T^{et}: 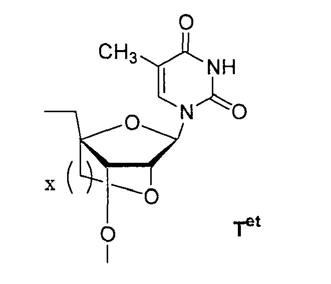 (where x represents an integer of 1 to 5);

R1 represents a hydroxyl group, a group represented by formula: 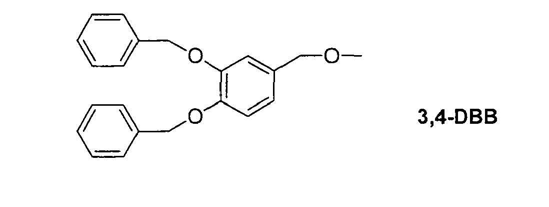 (hereinafter referred to as "3,4-DBB"), the group 3,4-DBB-(CH₂)₃-O-P(=O)(OH)-O-, the group 3,4-DBB-(CH₂)₃-O-P(=S)(OH)-O-, the group 3,4-DBB-(CH₂)₆-OP(=O)(OH)-O-, the group 3,4-DBB-(CH₂)₆-O-P(=S)(OH)-O-, the group C₁₅H₃₁C(O)O(CH₂)₂SP(=O)(OH)-O-, the group C₁₆H₃₃C(O)O(CH₂)₂SP(=O)(OH)-O-, the group C₁₇H₃₅C(O)O(CH₂)₂SP(=O)(OH)-O-, the group C₁₈H₃₇C(O)O(CH₂)₂SP(=O)(OH)-O-, or the group C₁₉H₃₉C(O)O(CH₂)₂SP(=O)(OH)-O-;

R2 represents a hydrogen atom, the group -P(=O)(OH)-O-CH₂-CH₂-OH, or the group -P(=S)(OH)-O-CH₂-CH₂-OH;

with the proviso that the following case is excluded:
B4, B5, and B8 are identical or different, and each is T^{p} or T^{s};
B1, B2, B3, and B12 are identical or different, and each is G^{p} or G^{s};
B16 is C^{p} or C^{s};
B6, B10, B 14, and B 18 are identical or different, and each is A^{p} or A^{s};
B11 is G^{t};
B15 is C^{t};
B9, B 13, B 17, and B 19 are each A^{t}; and
B7 is T^{t}),
or a pharmacologically acceptable salt thereof.

The above-described compound of formula (I) or a pharmacologically acceptable salt thereof is preferably:
(1) a compound wherein B4, B5, and B8 are identical or different, and each is T^{ep} or T^{es};
   B1, B2, B3, and B12 are identical or different, and each is G^{ep} or G^{es};
   B16 is C^{ep} or C^{es};
   B6, B 10, B 14, and B 18 are identical or different, and each is A^{ep} or A^{es};
   B11 is G^{et};
   B 15 is C^{et};
   B9, B 13, B 17, and B 19 are each A^{et}; and
   B7 is T^{et},
      or a pharmacologically acceptable salt thereof,
(2) a compound wherein B4, B5, and B8 are each T^{es};
   B1, B2, B3, and B12 are each G^{es};
   B16 is C^{es}; and
   B6, B10, B14, and B18 are each A^{es},
      or a pharmacologically acceptable salt thereof,
(3) a compound wherein E1 is a group represented by the formula R1-B5-B6-, or a pharmacologically acceptable salt thereof,
(4) a compound wherein E1 is a group represented by the formula R1-B6-, or a pharmacologically acceptable salt thereof,
(5) a compound wherein E1 is a group represented by the formula R1, or a pharmacologically acceptable salt thereof,
(6) a compound wherein E2 is a group represented by the formula -B8-B10-B12-B14-B16-B18-B19-R2, or a pharmacologically acceptable salt thereof,
(7) a compound wherein E2 is a group represented by the formula -B8-B10-B12-B14-B16-B17-R2, or a pharmacologically acceptable salt thereof,
(8) a compound wherein E2 is a group represented by the formula -B8-B10-B12-B14-B-15-R2, or a pharmacologically acceptable salt thereof,
(9) a compound wherein E2 is a group represented by the formula -B8-B10-B12-B13-R2, or a pharmacologically acceptable salt thereof,
(10) a compound wherein E2 is a group represented by the formula -B8-B10-B11-R2, or a pharmacologically acceptable salt thereof,
(11) a compound wherein E2 is a group represented by the formula -B8-B9-R2, or a pharmacologically acceptable salt thereof,
(12) a compound wherein E2 is a group represented by the formula -B7-R2, or a pharmacologically acceptable salt thereof,
(13) a compound wherein R1 is a hydroxyl group, or a pharmacologically acceptable salt thereof,
(14) a compound wherein R2 is the group -P(=O)(OH)-O-CH₂-CH₂-OH or the group -P(=S)(OH)-O-CH₂-CH₂-OH, or a pharmacologically acceptable salt thereof,
(15) a compound wherein R2 is the group -P(=S)(OH)-O-CH₂-CH₂-OH, or a pharmacologically acceptable salt thereof,
(16) a compound wherein each of l, m, n, o, p, q, r , s, t, u, v, w, and x is identical or different, and each is 1 or 2, or a pharmacologically acceptable salt thereof,
(17) a compound wherein 1, m, n, o, p, q, r , s, t, u, v, w, and x are identical, and each is 1 or 2, or a pharmacologically acceptable salt thereof, or
(18) a compound wherein each of 1, m, n, o, p, q, r , s, t, u, v, w, and x is 2, or a pharmacologically acceptable salt thereof.

More preferred is a compound selected from the following (compound group):
(compound group)
Illustrative Compound No. 1: HO-T^{e2s}-A^{e2s}-G^{e2s}-G^{e2s}-G^{e2s}-T^{e2s}-T^{e2s}-A^{e2s}-G^{e2s}-A^{e2s}-C^{e2s}-A^{e2s}-A^{e2s}-CH₂CH₂OH
Illustrative Compound No. 2: HO-T^{e2s}-A^{e2s}-G^{e2s}-G^{e2s}-G^{e2s}-T^{e2s}-T^{e2s}-A^{e2s}-G^{e2s}-A^{e2s}-C^{e2s}-A^{e2s}-CH₂CH₂OH
Illustrative Compound No. 3: HO-T^{e2s}-A^{e2s}-G^{e2s}-G^{e2s}-G^{e2s}-T^{e2s}-T^{e2s}-A^{e2s}-G^{e2s}-A^{e2s}-C^{e2s}-CH₂CH₂OH
Illustrative Compound No. 4: HO-T^{e2s}-A^{e2s}-G^{e2s}-G^{e2s}-G^{e2s}-T^{e2s}-T^{e2s}-A^{e2s}-G^{e2s}-A^{e2s}-CH₂CH₂OH
Illustrative Compound No. 5: HO-T^{e2s}-A^{e2s}-G^{e2s}-G^{e2s}-G^{e2s}-T^{e2s}-T^{e2s}-A^{e2s}-G^{e2s}-CH₂CH₂OH
Illustrative Compound No. 6: HO-T^{e2s}-A^{e2s}-G^{e2s}-G^{e2s}-G^{e2s}-T^{e2s}-T^{e2s}-A^{e2s}-CH₂CH₂OH
Illustrative Compound No. 7: HO-T^{e2s}-A^{e2s}-G^{e2s}-G^{e2s}-G^{e2s}-T^{e22s}-T^{e2s}-CH₂CH₂OH
Illustrative Compound No. 8: HO-A^{e2s}-G^{e2s}-G^{e2s}-G^{e2s}-T^{e2s}-T^{e2s}-A^{e2s}-G^{e2s}-A^{e2s}-C^{e2s}-A^{e2s}-A^{e2s}-CH₂CH₂OH
Illustrative Compound No. 9: HO-A^{e2s}-G^{e2s}-G^{e2s}-G^{e2s}-T^{e2s}-T^{e2s}-A^{e2s}-G^{e2s}-A^{e2s}-C^{e2s}-A^{e2s}-CH₂CH₂OH
Illustrative Compound No. 10: HO-A^{e2s}-G^{e2s}-G^{e2s}-G^{e2s}-T^{e2s}-T^{e2s}-A^{e2s}-G^{e2s}-A^{e2s}-C^{e2s}-CH₂CH₂OH
Illustrative Compound No. 11: HO-A^{e2s}-G^{e2s}-G^{e2s}-G^{e2s}-T^{e2s}-T^{e2s}-A^{e2s}-G^{e2s}-A^{e2s}-CH₂CH₂OH
Illustrative Compound No. 12: HO-A^{e2s}-G^{e2s}-G^{e2s}-G^{e2s}-T^{e2s}-T^{e2s}-A^{e2s}-G^{e2s}-CH₂CH₂OH
Illustrative Compound No. 13: HO-A^{e2s}-G^{e2s}-G^{e2s}-G^{e2s}-T^{e2s}-T^{e2s}-A^{e2s}-CH₂CH₂OH
Illustrative Compound No. 14: HO-G^{e2s}-G^{e2s}-G^{e2s}-T^{e2s}-T^{e2s}-A^{e2s}-G^{e2s}-A^{e2s}-C^{e2s}-A^{e2s}-A^{e2s}-CH₂CH₂OH
Illustrative Compound No. 15: HO-G^{e2s}-G^{e2s}-G^{e2s}-T^{e2s}-T^{e2s}-A^{e2s}-G^{e2s}-A^{e2s}-C^{e2s}-A^{e2s}-CH₂CH₂OH
Illustrative Compound No. 16: HO-G^{e2s}-G^{e2s}-G^{e2s}-T^{e2s}-T^{e2s}-A^{e2s}-G^{e2s}-A^{e2s}-C^{e2s}-CH₂CH₂OH
Illustrative Compound No. 17: HO-G^{e2s}-G^{e2s}-G^{e2s}-T^{e2s}-T^{e2s}-A^{e2s}-G^{e2s}-A^{e2s}-CH₂CH₂OH
Illustrative Compound No. 18: HO-G^{e2s}-G^{e2s}-G^{e2s}-T^{e2s}-T^{e2s}-A^{e2s}-G^{e2s}-CH₂CH₂OH
Illustrative Compound No. 19: HO-T^{e2s}-A^{e2s}-G^{e2s}-G^{e2s}-G^{e2s}-T^{e2s}-T^{e2s}-A^{e2s}-G^{e2s}-A^{e2s}-C^{e2s}-A^{e2s}-A^{e2t}-H
Illustrative Compound No. 20: HO-T^{e2s}-A^{e2s}-G^{e2s}-G^{e2s}-G^{e2s}-T^{e2s}-T^{e2s}-A^{e2s}-G^{e2s}-A^{e2s}-C^{e2s}-A^{e2t}-H
Illustrative Compound No. 21: HO-T^{e2s}-A^{e2s}-G^{e2s}-G^{e2s}-G^{e2s}-T^{e2s}-T^{e2s}-A^{e2s}-G^{e2s}-A^{e2s}-C^{e2t}-H
Illustrative Compound No. 22: HO-T^{e2s}-A^{e2s}-G^{e2s}-G^{e2s}-G^{e2s}-T^{e2s}-T^{e2s}-A^{e2s}-G^{e2s}-A^{e2t}-H
Illustrative Compound No. 23: HO-T^{e2s}-A^{e2s}-G^{e2s}-G^{e2s}-G^{e2s}-T^{e2s}-T^{e2s}-A^{e2s}-G^{e2t}-H
Illustrative Compound No. 24: HO-T^{e2s}-A^{e2s}-G^{e2s}-G^{e2s}-G^{e2s}-T^{e2s}-T^{e2s}-A^{e2t}-H
Illustrative Compound No. 25: HO-T^{e2s}-A^{e2s}-G^{e2s}-G^{e2s}-G^{e2s}-T^{e2s}-T^{e2t}-H
Illustrative Compound No. 26: HO-A^{e2s}-G^{e2s}-G^{e2s}-G^{e2s}-T^{e2s}-T^{e2s}-A^{e2s}-G^{e2s}-A^{e2s}-C^{e2s}-A^{e2s}-A^{e2t}-H
Illustrative Compound No. 27: HO-A^{e2s}-G^{e2s}-G^{e2s}-G^{e2s}-T^{e2s}-T^{e2s}-A^{e2s}-G^{e2s}-A^{e2s}-C^{e2s}-A^{e2t}-H
Illustrative Compound No. 28: HO-A^{e2s}-G^{e2s}-G^{e2s}-G^{e2s}-T^{e2s}-T^{e2s}-A^{e2s}-G^{e2s}-A^{e2s}-C^{e2t}-H
Illustrative Compound No. 29: HO-A^{e2s}-G^{e2s}-G^{e2s}-G^{e2s}-T^{e2s}-T^{e2s}-A^{e2s}-G^{e2s}-A^{e2t}-H
Illustrative Compound No. 30: HO-A^{e2s}-G^{e2s}-G^{e2s}-G^{e2s}-T^{e2s}-T^{e2s}-A^{e2s}-G^{e2t}-H
Illustrative Compound No. 31: HO-A^{e2s}-G^{e2s}-G^{e2s}-G^{e2s}-T^{e2s}-T^{e2s}-A^{e2t}-H
Illustrative Compound No. 32: HO-G^{e2s}-G^{e2s}-G^{e2s}-T^{e2s}-T^{e2s}-A^{e2s}-G^{e2s}-A^{e2s}-C^{e2s}-A^{e2s}-A^{e2t}-H
Illustrative Compound No. 33: HO-G^{e2s}-G^{e2s}-G^{e2s}-T^{e2s}-T^{e2s}-A^{e2s}-G^{e2s}-A^{e2s}-C^{e2s}-A^{e2t}-H
Illustrative Compound No. 34: HO-G^{e2s}-G^{e2s}-G^{e2s}-T^{e2s}-T^{e2s}-A^{e2s}-G^{e2s}-A^{e2s}-C^{e2t}-H
Illustrative Compound No. 35: HO-G^{e2s}-G^{e2s}-G^{e2s}-T^{e2s}-T^{e2s}-A^{e2s}-G^{e2s}-A^{e2t}-H
Illustrative Compound No. 36: HO-G^{e2s}-G^{e2s}-G^{e2s}-T^{e2s}-T^{e2s}-A^{e2s}-G^{e2t}-H
Illustrative Compound No. 37: HO-T^{e2p}-A^{e2p}-G^{e2p}-G^{e2p}-G^{e2p}-T^{e2p}-T^{e2p}-A^{e2p}-G^{e2p}-A^{e2p}-C^{e2p}-A^{e2p}-A^{e2p}-CH₂CH₂OH
Illustrative Compound No. 38: HO-T^{e2p}-A^{e2p}-G^{e2p}-G^{e2p}-G^{e2p}-T^{e2p}-T^{e2p}-A^{e2p}-G^{e2p}-A^{e2p}-C^{e2p}-A^{e2p}-CH₂CH₂OH
Illustrative Compound No. 39: HO-T^{e2p}-A^{e2p}-G^{e2p}-G^{e2p}-G^{e2p}-T^{e2p}-T^{e2p}-A^{e2p}-G^{e2p}-A^{e2p}-C^{e2p}-CH₂CH₂OH
Illustrative Compound No. 40: HO-T^{e2p}-A^{e2p}-G^{e2p}-G^{e2p}-G^{e2p}-T^{e2p}-T^{e2p}-A^{e2p}-G^{e2p}-A^{e2p}-CH₂CH₂OH
Illustrative Compound No. 41: HO-T^{e2p}-A^{e2p}-G^{e2p}-G^{e2p}-G^{e2p}-T^{e2p}-T^{e2p}-A^{e2p}-G^{e2p}-CH₂CH₂OH
Illustrative Compound No. 42: HO-T^{e2p}-A^{e2p}-G^{e2p}-G^{e2p}-G^{e2p}-T^{e2p}-T^{e2p}-A^{e2p}-CH₂CH₂OH
Illustrative Compound No. 43: HO-T^{e2p}-A^{e2p}-G^{e2p}-G^{e2p}-G^{e2p}-T^{e2p}-T^{e2p}-CH₂CH₂OH
Illustrative Compound No. 44: HO-A^{e2p}-G^{e2p}-G^{e2p}-G^{e2p}-T^{e2p}-T^{e2p}-A^{e2p}-G^{e2p}-A^{e2p}-C^{e2p}-A^{e2p}-A^{e2p}-CH₂CH₂OH
Illustrative Compound No. 45: HO-A^{e2p}-G^{e2p}-G^{e2p}-G^{e2p}-T^{e2p}-T^{e2p}-A^{e2p}-G^{e2p}-A^{e2p}-C^{e2p}-A^{e2p}-CH₂CH₂OH
Illustrative Compound No. 46: HO-A^{e2p}-G^{e2p}-G^{e2p}-G^{e2p}-T^{e2p}-T^{e2p}-A^{e2p}-G^{e2p}-A^{e2p}-C^{e2p}-CH₂CH₂OH
Illustrative Compound No. 47: HO-A^{e2p}-G^{e2p}-G^{e2p}-G^{e2p}-T^{e2p}-T^{e2p}-A^{e2p}-G^{e2p}-A^{e2p}-CH₂CH₂OH
Illustrative Compound No. 48: HO-A^{e2p}-G^{e2p}-G^{e2p}-G^{e2p}-T^{e2p}-T^{e2p}-A^{e2p}-G^{e2p}-CH₂CH₂OH
Illustrative Compound No. 49: HO-A^{e2p}-G^{e2p}-G^{e2p}-G^{e2p}-T^{e2p}-T^{e2p}-A^{e2p}-CH₂CH₂OH
Illustrative Compound No. 50: HO-G^{e2p}-G^{e2p}-G^{e2p}-T^{e2p}-T^{e2p}-A^{e2p}-G^{e2p}-A^{e2p}-C^{e2p}-A^{e2p}-A^{e2p}-CH₂CH₂OH
Illustrative Compound No. 51: HO-G^{e2p}-G^{e2p}-G^{e2p}-T^{e2p}-T^{e2p}-A^{e2p}-G^{e2p}-A^{e2p}-C^{e2p}-A^{e2p}-CH₂CH₂OH
Illustrative Compound No. 52: HO-G^{e2p}-G^{e2p}-G^{e2p}-T^{e2p}-T^{e2p}-A^{e2p}-G^{e2p}-A^{e2p}-C^{e2p}-CH₂CH₂OH
Illustrative Compound No. 53: HO-G^{e2p}-G^{e2p}-G^{e2p}-T^{e2p}-T^{e2p}-A^{e2p}-G^{e2p}-A^{e2p}-CH₂CH₂OH
Illustrative Compound No. 54: HO-G^{e2p}-G^{e2p}-G^{e2p}-T^{e2p}-T^{e2p}-A^{e2p}-G^{e2p}-CH₂CH₂OH
Illustrative Compound No. 55: HO-T^{e2p}-A^{e2p}-G^{e2p}-G^{e2p}-G^{e2p}-T^{e2p}-T^{e2p}-A^{e2p}-G^{e2p}-A^{e2p}-C^{e2p}-A^{e2p}-A^{e2t}-H
Illustrative Compound No. 56: HO-T^{e2p}-A^{e2p}-G^{e2p}-G^{e2p}-G^{e2p}-T^{e2p}-T^{e2p}-A^{e2p}-G^{e2p}-A^{e2p}-C^{e2p}-A^{e2t}-H
Illustrative Compound No. 57: HO-T^{e2p}-A^{e2p}-G^{e2p}-G^{e2p}-G^{e2p}-T^{e2p}-T^{e2p}-A^{e2p}-G^{e2p}-A^{e2p}-C^{e2t}-H
Illustrative Compound No. 58: HO-T^{e2p}-A^{e2p}-G^{e2p}-G^{e2p}-G^{e2p}-T^{e2p}-T^{e2p}-A^{e2p}-G^{e2p}-A^{e2t}-H
Illustrative Compound No. 59: HO-T^{e2p}-A^{e2p}-G^{e2p}-G^{e2p}-G^{e2p}-T^{e2p}-T^{e2p}-A^{e2p}-G^{e2t}-H
Illustrative Compound No. 60: HO-T^{e2p}-A^{e2p}-G^{e2p}-G^{e2p}-G^{e2p}-T^{e2p}-T^{e2p}-A^{e2t}-H
Illustrative Compound No. 61: HO-T^{e2p}-A^{e2p}-G^{e2p}-G^{e2p}-G^{e2p}-T^{e2p}-T^{e2t}-H
Illustrative Compound No. 62: HO-A^{e2p}-G^{e2p}-G^{e2p}-G^{e2p}-T^{e2p}-T^{e2p}-A^{e2p}-G^{e2p}-A^{e2p}-C^{e2p}-A^{e2p}-A^{e2t}-H
Illustrative Compound No. 63: HO-A^{e2p}-G^{e2p}-G^{e2p}-G^{e2p}-T^{e2p}-T^{e2p}-A^{e2p}-G^{e2p}-A^{e2p}-C^{e2p}-A^{e2t}-H
Illustrative Compound No. 64: HO-A^{e2p}-G^{e2p}-G^{e2p}-G^{e2p}-T^{e2p}-T^{e2p}-A^{e2p}-G^{e2p}-A^{e2p}-C^{e2t}-H
Illustrative Compound No. 65: HO-A^{e2p}-G^{e2p}-G^{e2p}-G^{e2p}-T^{e2p}-T^{e2p}-A^{e2p}-G^{e2p}-A^{e2t}-H
Illustrative Compound No. 66: HO-A^{e2p}-G^{e2p}-G^{e2p}-G^{e2p}-T^{e2p}-T^{e2p}-A^{e2p}-G^{e2t}-H
Illustrative Compound No. 67: HO-A^{e2p}-G^{e2p}-G^{e2p}-G^{e2p}-T^{e2p}-T^{e2p}-A^{e2t}-H
Illustrative Compound No. 68: HO-G^{e2p}-G^{e2p}-G^{e2p}-T^{e2p}-T^{e2p}-A^{e2p}-G^{e2p}-A^{e2p}-C^{e2p}-A^{e2p}-A^{e2t}-H
Illustrative Compound No. 69: HO-G^{e2p}-G^{e2p}-G^{e2p}-T^{e2p}-T^{e2p}-A^{e2p}-G^{e2p}-A^{e2p}-C^{e2p}-A^{e2t}-H
Illustrative Compound No. 70: HO-G^{e2p}-G^{e2p}-G^{e2p}-T^{e2p}-T^{e2p}-A^{e2p}-G^{e2p}-A^{e2p}-C^{e2t}-H
Illustrative Compound No. 71: HO-G^{e2p}-G^{e2p}-G^{e2p}-T^{e2p}-T^{e2p}-A^{e2p}-G^{e2p}-A^{e2t}-H
Illustrative Compound No. 72: HO-G^{e2p}-G^{e2p}-G^{e2p}-T^{e2p}-T^{e2p}-A^{e2p}-G^{e2t}-H
Illustrative Compound No. 73: HO-T^{e1s}-A^{e1s}-G^{e1s}-G^{e1s}-G^{e1s}-T^{e1s}-T^{e1s}-A^{e1s}-G^{e1s}-A^{e1s}-C^{e1s}-A^{e1s}-A^{e1s}-CH₂CH₂OH
Illustrative Compound No. 74: HO-T^{e1s}-A^{e1s}-G^{e1s}-G^{e1s}-G^{e1s}-T^{e1s}-T^{e1s}-A^{e1s}-G^{e1s}-A^{e1s}-C^{e1s}-A^{e1s}-CH₂CH₂OH
Illustrative Compound No. 75: HO-T^{e1s}-A^{e1s}-G^{e1s}-G^{e1s}-G^{e1s}-T^{e1s}-T^{e1s}-A^{e1s}-G^{e1s}-A^{e1s}-C^{e1s}-CH₂CH₂OH
Illustrative Compound No. 76: HO-T^{e1s}-A^{e1s}-G^{e1s}-G^{e1s}-G^{e1s}-T^{e1s}-T^{e1s}-A^{e1s}-G^{e1s}-A^{e1s}-CH₂CH₂OH
Illustrative Compound No. 77: HO-T^{e1s}-A^{e1s}-G^{e1s}-G^{e1s}-G^{e1s}-T^{e1s}-T^{e1s}-A^{e1s}-G^{e1s}-CH₂CH₂OH
Illustrative Compound No. 78: HO-T^{e1s}-A^{e1s}-G^{e1s}-G^{e1s}-G^{e1s}-T^{e1s}-T^{e1s}-A^{e1s}-CH₂CH₂OH
Illustrative Compound No. 79: HO-T^{e1s}-A^{e1s}-G^{e1s}-G^{e1s}-G^{e1s}-T^{e1s}-T^{e1s}-CH₂CH₂OH
Illustrative Compound No. 80: HO-A^{e1s}-G^{e1s}-G^{e1s}-G^{e1s}-T^{e1s}-T^{e1s}-A^{e1s}-G^{e1s}-A^{e1s}-C^{e1s}-A^{e1s}-A^{e1s}-CH₂CH₂OH
Illustrative Compound No. 81: HO-A^{e1s}-G^{e1s}-G^{e1s}-G^{e1s}-T^{e1s}-T^{e1s}-A^{e1s}-G^{e1s}-A^{e1s}-C^{e1s}-A^{e1s}-CH₂CH₂OH
Illustrative Compound No. 82: HO-A^{e1s}-G^{e1s}-G^{e1s}-G^{e1s}-T^{e1s}-T^{e1s}-A^{e1s}-G^{e1s}-A^{e1s}-C^{e1s}-CH₂CH₂OH
Illustrative Compound No. 83: HO-A^{e1s}-G^{e1s}-G^{e1s}-G^{e1s}-T^{e1s}-T^{e1s}-A^{e1s}-G^{e1s}-A^{e1s}-CH₂CH₂OH
Illustrative Compound No. 84: HO-A^{e1s}-G^{e1s}-G^{e1s}-G^{e1s}-T^{e1s}-T^{e1s}-A^{e1s}-G^{e1s}-CH₂CH₂OH
Illustrative Compound No. 85: HO-A^{e1s}-G^{e1s}-G^{e1s}-G^{e1s}-T^{e1s}-T^{e1s}-A^{e1s}-CH₂CH₂OH
Illustrative Compound No. 86: HO-G^{e1s}-G^{e1s}-G^{e1s}-T^{e1s}-T^{e1s}-A^{e1s}-G^{e1s}-A^{e1s}-C^{e1s}-A^{e1s}-A^{e1s}-CH₂CH₂OH
Illustrative Compound No. 87: HO-G^{e1s}-G^{e1s}-G^{e1s}-T^{e1s}-T^{e1s}-A^{e1s}-G^{e1s}-A^{e1s}-C^{e1s}-A^{e1s}-CH₂CH₂OH
Illustrative Compound No. 88: HO-G^{e1s}-G^{e1s}-G^{e1s}-T^{e1s}-T^{e1s}-A^{e1s}-G^{e1s}-A^{e1s}-C^{e1s}-CH₂CH₂OH
Illustrative Compound No. 89: HO-G^{e1s}-G^{e1s}-G^{e1s}-T^{e1s}-T^{e1s}-A^{e1s}-G^{e1s}-A^{e1s}-CH₂CH₂OH
Illustrative Compound No. 90: HO-G^{e1s}-G^{e1s}-G^{e1s}-T^{e1s}-T^{e1s}-A^{e1s}-G^{e1s}-CH₂CH₂OH
Illustrative Compound No. 91: HO-T^{e1s}-A^{e1s}-G^{e1s}-G^{e1s}-G^{e1s}-T^{e1s}-T^{e1s}-A^{e1s}-G^{e1s}-A^{e1s}-C^{e1s}-A^{e1s}-A^{e1t}-H
Illustrative Compound No. 92: HO-T^{e1s}-A^{e1s}-G^{e1s}-G^{e1s}-G^{e1s}-T^{e1s}-T^{e1s}-A^{e1s}-G^{e1s}-A^{e1s}-C^{e1s}-A^{e1t}-H
Illustrative Compound No. 93: HO-T^{e1s}-A^{e1s}-G^{e1s}-G^{e1s}-G^{e1s}-T^{e1s}-T^{e1s}-A^{e1s}-G^{e1s}-A^{e1s}-C^{e1t}-H
Illustrative Compound No. 94: HO-T^{e1s}-A^{e1s}-G^{e1s}-G^{e1s}-G^{e1s}-T^{e1s}-T^{e1s}-A^{e1s}-G^{e1s}-A^{e1t}-H
Illustrative Compound No. 95: HO-T^{e1s}-A^{e1s}-G^{e1s}-G^{e1s}-G^{e1s}-T^{e1s}-T^{e1s}-A^{e1s}-G^{e1t}-H
Illustrative Compound No. 96: HO-T^{e1s}-A^{e1s}-G^{e1s}-G^{e1s}-G^{e1s}-T^{e1s}-T^{e1s}-A^{e1t}-H
Illustrative Compound No. 97: HO-T^{e1s}-A^{e1s}-G^{e1s}-G^{e1s}-G^{e1s}-T^{e1s}-T^{e1t}-H
Illustrative Compound No. 98: HO-A^{e1s}-G^{e1s}-G^{e1s}-G^{e1s}-T^{e1s}-T^{e1s}-A^{e1s}-G^{e1s}-A^{e1s}-C^{e1s}-A^{e1s}-A^{e1t}-H
Illustrative Compound No. 99: HO-A^{e1s}-G^{e1s}-G^{e1s}-G^{e1s}-T^{e1s}-T^{e1s}-A^{e1s}-G^{e1s}-A^{e1s}-C^{e1s}-A^{e1t}-H
Illustrative Compound No. 100: HO-A^{e1s}-G^{e1s}-G^{e1s}-G^{e1s}-T^{e1s}-T^{e1s}-A^{e1s}-G^{e1s}-A^{e1s}-C^{e1t}-H
Illustrative Compound No. 101: HO-A^{e1s}-G^{e1s}-G^{e1s}-G^{e1s}-T^{e1s}-T^{e1s}-A^{e1s}-G^{e1s}-A^{e1t}-H
Illustrative Compound No. 102: HO-A^{e1s}-G^{e1s}-G^{e1s}-G^{e1s}-T^{e1s}-T^{e1s}-A^{e1s}-G^{e1t}-H.
Illustrative Compound No. 103: HO-A^{e1s}-G^{e1s}-G^{e1s}-G^{e1s}-T^{e1s}-T^{e1s}-A^{e1t}-H
Illustrative Compound No. 104: HO-G^{e1s}-G^{e1s}-G^{e1s}-T^{e1s}-T^{e1s}-A^{e1s}-G^{e1s}-A^{e1s}-C^{e1s}-A^{e1s}-A^{e1t}-H
Illustrative Compound No. 105: HO-G^{e1s}-G^{e1s}-G^{e1s}-T^{e1s}-T^{e1s}-A^{e1s}-G^{e1s}-A^{e1s}-C^{e1s}-A^{e1t}-H
Illustrative Compound No. 106: HO-G^{e1s}-G^{e1s}-G^{e1s}-T^{e1s}-T^{e1s}-A^{e1s}-G^{e1s}-A^{e1s}-C^{e1t}-H
Illustrative Compound No. 107: HO-G^{e1s}-G^{e1s}-G^{e1s}-T^{e1s}-T^{e1s}-A^{e1s}-G^{e1s}-A^{e1t}-H
Illustrative Compound No. 108: HO-G^{e1s}-G^{e1s}-G^{e1s}-T^{e1s}-T^{e1s}-A^{e1s}-G^{e1t}-H
Illustrative Compound No. 109: HO-T^{e1p}-A^{e1p}-G^{e1p}-G^{e1p}-G^{e1p}-T^{e1p}-T^{e1p}-A^{e1p}-G^{e1p}-A^{e1p}-C^{e1p}-A^{e1p}-A^{e1p}-CH₂CH₂OH
Illustrative Compound No. 110: HO-T^{e1p}-A^{e1p}-G^{e1p}-G^{e1p}-G^{e1p}-T^{e1p}-T^{e1p}-A^{e1p}-G^{e1p}-A^{e1p}-C^{e1p}-A^{e1p}-CH₂CH₂OH
Illustrative Compound No. 111: HO-T^{e1p}-A^{e1p}-G^{e1p}-G^{e1p}-G^{e1p}-T^{e1p}-T^{e1p}-A^{e1p}-G^{e1p}-A^{e1p}-C^{e1p}-CH₂CH₂OH
Illustrative Compound No. 112: HO-T^{e1p}-A^{e1p}-G^{e1p}-G^{e1p}-G^{e1p}-T^{e1p}-T^{e1p}-A^{e1p}-G^{e1p}-A^{e1p}-CH₂CH₂OH
Illustrative Compound No. 113: HO-T^{e1p}-A^{e1p}-G^{e1p}-G^{e1p}-G^{e1p}-T^{e1p}-T^{e1p}-A^{e1p}-G^{e1p}-CH₂CH₂OH
Illustrative Compound No. 114: HO-T^{e1p}-A^{e1p}-G^{e1p}-G^{e1p}-G^{e1p}-T^{e1p}-T^{e1p}-A^{e1p}-CH₂CH₂OH
Illustrative Compound No. 115: HO-T^{e1p}-A^{e1p}-G^{e1p}-G^{e1p}-G^{e1p}-T^{e1p}-T^{e1p}-CH₂CH₂OH
Illustrative Compound No. 116: HO-A^{e1p}-G^{e1p}-G^{e1p}-G^{e1p}-T^{e1p}-T^{e1p}-A^{e1p}-G^{e1p}-A^{e1p}-C^{e1p}-A^{e1p}-A^{e1p}-CH₂CH₂OH
Illustrative Compound No. 117: HO-A^{e1p}-G^{e1p}-G^{e1p}-G^{e1p}-T^{e1p}-T^{e1p}-A^{e1p}-G^{e1p}-A^{e1p}-C^{e1p}-A^{e1p}-CH₂CH₂OH
Illustrative Compound No. 118: HO-A^{e1p}-G^{e1p}-G^{e1p}-G^{e1p}-T^{e1p}-T^{e1p}-A^{e1p}-G^{e1p}-A^{e1p}-C^{e1p}-CH₂CH₂OH
Illustrative Compound No. 119: HO-A^{e1p}-G^{e1p}-G^{e1p}-G^{e1p}-T^{e1p}-T^{e1p}-A^{e1p}-G^{e1p}-A^{e1p}-CH₂CH₂OH
Illustrative Compound No. 120: HO-A^{e1p}-G^{e1p}-G^{e1p}-G^{e1p}-T^{e1p}-T^{e1p}-A^{e1p}-G^{e1p}-CH₂CH₂OH
Illustrative Compound No. 121: HO-A^{e1p}-G^{e1p}-G^{e1p}-G^{e1p}-T^{e1p}-T^{e1p}-A^{e1p}-CH₂CH₂OH
Illustrative Compound No. 122: HO-G^{e1p}-G^{e1p}-G^{e1p}-T^{e1p}-T^{e1p}-A^{e1p}-G^{e1p}-A^{e1p}-C^{e1p}-A^{e1p}-A^{e1p}-CH₂CH₂OH
Illustrative Compound No. 123: HO-G^{e1p}-G^{e1p}-G^{e1p}-T^{e1p}-T^{e1p}-A^{e1p}-G^{e1p}-A^{e1p}-C^{e1p}-A^{e1p}-CH₂CH₂OH
Illustrative Compound No. 124: HO-G^{e1p}-G^{e1p}-G^{e1p}-T^{e1p}-T^{e1p}-A^{e1p}-G^{e1p}-A^{e1p}-C^{e1p}-CH₂CH₂OH
Illustrative Compound No. 125: HO-G^{e1p}-G^{e1p}-G^{e1p}-T^{e1p}-T^{e1p}-A^{e1p}-G^{e1p}-A^{e1p}-CH₂CH₂OH
Illustrative Compound No. 126: HO-G^{e1p}-G^{e1p}-G^{e1p}-T^{e1p}-T^{e1p}-A^{e1p}-G^{e1p}-CH₂CH₂OH
Illustrative Compound No. 127: HO-T^{e1p}-A^{e1p}-G^{e1p}-G^{e1p}-G^{e1p}-T^{e1p}-T^{e1p}-A^{e1p}-G^{e1p}-A^{e1p}-C^{e1p}-A^{e1p}-A^{e1t}-H
Illustrative Compound No. 128: HO-T^{e1p}-A^{e1p}-G^{e1p}-G^{e1p}-G^{e1p}-T^{e1p}-T^{e1p}-A^{e1p}-G^{e1p}-A^{e1p}-C^{e1p}-A^{e1t}-H
Illustrative Compound No. 129: HO-T^{e1p}-A^{e1p}-G^{e1p}-G^{e1p}-G^{e1p}-T^{e1p}-T^{e1p}-A^{e1p}-G^{e1p}-A^{e1p}-C^{e1t}-H
Illustrative Compound No. 130: HO-T^{e1p}-A^{e1p}-G^{e1p}-G^{e1p}-G^{e1p}-T^{e1p}-T^{e1p}-A^{e1p}-G^{e1p}-A^{e1t}-H
Illustrative Compound No. 131: HO-T^{e1p}-A^{e1p}-G^{e1p}-G^{e1p}-G^{e1p}-T^{e1p}-T^{e1p}-A^{e1p}-G^{e1t}-H
Illustrative Compound No. 132: HO-T^{e1p}-A^{e1p}-G^{e1p}-G^{e1p}-G^{e1p}-T^{e1p}-T^{e1p}-A^{e1t}-H
Illustrative Compound No. 133: HO-T^{e1p}-A^{e1p}-G^{e1p}-G^{e1p}-G^{e1p}-T^{e1p}-T^{e1t}-H
Illustrative Compound No. 134: HO-A^{e1p}-G^{e1p}-G^{e1p}-G^{e1p}-T^{e1p}-T^{e1p}-A^{e1p}-G^{e1p}-A^{e1p}-C^{e1p}-A^{e1p}-A^{e1t}-H
Illustrative Compound No. 135: HO-A^{e1p}-G^{e1p}-G^{e1p}-G^{e1p}-T^{e1p}-T^{e1p}-A^{e1p}-G^{e1p}-A^{e1p}-C^{e1p}-A^{e1t}-H
Illustrative Compound No. 136: HO-A^{e1p}-G^{e1p}-G^{e1p}-G^{e1p}-T^{e1p}-T^{e1p}-A^{e1p}-G^{e1p}-A^{e1p}-C^{e1t}-H
Illustrative Compound No. 137: HO-A^{e1p}-G^{e1p}-G^{e1p}-G^{e1p}-T^{e1p}-T^{e1p}-A^{e1p}-G^{e1p}-A^{e1t}-H
Illustrative Compound No. 138: HO-A^{e1p}-G^{e1p}-G^{e1p}-G^{e1p}-T^{e1p}-T^{e1p}-A^{e1p}-G^{e1t}-H
Illustrative Compound No. 139: HO-A^{e1p}-G^{e1p}-G^{e1p}-G^{e1p}-T^{e1p}-T^{e1p}-A^{e1t}-H
Illustrative Compound No. 140: HO-G^{e1p}-G^{e1p}-G^{e1p}-T^{e1p}-T^{e1p}-A^{e1p}-G^{e1p}-A^{e1p}-C^{e1p}-A^{e1p}-A^{e1t}-H
Illustrative Compound No. 141: HO-G^{e1p}-G^{e1p}-G^{e1p}-T^{e1p}-T^{e1p}-A^{e1p}-G^{e1p}-A^{e1p}-C^{e1p}-A^{e1t}-H
Illustrative Compound No. 142: HO-G^{e1p}-G^{e1p}-G^{e1p}-T^{e1p}-T^{e1p}-A^{e1p}-G^{e1p}-A^{e1p}-C^{e1t}-H
Illustrative Compound No. 143: HO-G^{e1p}-G^{e1p}-G^{e1p}-T^{e1p}-T^{e1p}-A^{e1p}-G^{e1p}-A^{e1t}-H
Illustrative Compound No. 144: HO-G^{e1p}-G^{e1p}-G^{e1p}-T^{e1p}-T^{e1p}-A^{e1p}-G^{e1t}-H
Illustrative Compound No. 145: 3,4-DBB-(CH₂)₃-O-P(=S)(OH)-O-T^{e2s}-A^{e2s}-G^{e2s}-G^{e2s}-G^{e2s}-T^{e2s}-T^{e2s}-A^{e2s}-G^{e2s}-A^{e2s}-C^{e2s}-A^{e2s}-A^{e2s}-CH₂CH₂OH
Illustrative Compound No. 146: 3,4-DBB-(CH₂)₃-O-P(=S)(OH)-O-T^{e2s}-A^{e2s}-G^{e2s}-G^{e2s}-G^{e2s}-T^{e2s}-T^{e2s}-A^{e2s}-G^{e2s}-A^{e2s}-C^{e2s}-A^{e2s}-A^{e2t}-H
Illustrative Compound No. 147: 3,4-DBB-(CH₂)₆-O-P(=S)(OH)-O-T^{e2s}-A^{e2s}-G^{e2s}-G^{e2s}-G^{e2s}-T^{e2s}-T^{e2s}-A^{e2s}-G^{e2s}-A^{e2s}-C^{e2s}-A^{e2s}-A^{e2s}-CH₂CH₂OH
Illustrative Compound No. 148: 3,4-DBB-(CH₂)₆-O-P(=S)(OH)-O-T^{e2s}-A^{e2s}-G^{e2s}-G^{e2s}-G^{e2s}-T^{e2s}-T^{e2s}-A^{e2s}-G^{e2s}-A^{e2s}-C^{e2s}-A^{e2s}-A^{e2t}-H
Illustrative Compound No. 149: 3,4-DBB-T^{s}-T^{e2s}-A^{e2s}-G^{e2s}-G^{e2s}-G^{e2s}-T^{e2s}-T^{e2s}-A^{e2s}-G^{e2s}-A^{e2s}-C^{e2s}-A^{e2s}-A^{e2s}-CH₂CH₂OH
Illustrative Compound No. 150: 3,4-DBB-T^{s}-T^{e2s}-A^{e2s}-G^{e2s}-G^{e2s}-G^{e2s}-T^{e2s}-T^{e2s}-A^{e2s}-G^{e2s}-A^{e2s}-C^{e2s}-A^{e2s}-A^{e2t}-H
Illustrative Compound No. 151: 3,4-DBB-T^{s}-A^{e2s}-G^{e2s}-G^{e2s}-G^{e2s}-T^{e2s}-T^{e2s}-A^{e2s}-G^{e2s}-A^{e2s}-C^{e2s}-A^{e2s}-A^{e2s}-CH₂CH₂OH
Illustrative Compound No. 152: 3,4-DBB-T^{s}-A^{e2s}-G^{e2s}-G^{e2s}-G^{e2s}-T^{e2s}-T^{e2s}-A^{e2s}-G^{e2s}-A^{e2s}-C^{e2s}-A^{e2s}-A^{e2t}-H
Illustrative Compound No. 153: HO-T^{e2s}-A^{e2s}-G^{e2p}-G^{e2p}-G^{e2p}-T^{e2p}-T^{e2p}-A^{e2p}-G^{e2p}-A^{e2p}-C^{e2p}-A^{e2s}-A^{e2s}-CH₂CH₂OH
Illustrative Compound No. 154: HO-T^{e2s}-A^{e2s}-G^{e2p}-G^{e2p}-G^{e2p}-T^{e2p}-T^{e2p}-A^{e2p}-G^{e2p}-A^{e2p}-C^{e2p}-A^{e2s}-A^{e2t}-H
Illustrative Compound No. 155: HO-T^{e2s}-A^{e2s}-G^{e2s}-G^{e2p}-G^{e2p}-T^{e2p}-T^{e2p}-A^{e2p}-G^{e2p}-A^{e2p}-C^{e2s}-A^{e2s}-A^{e2s}-CH₂CH₂OH
Illustrative Compound No. 156: HO-T^{e2s}-A^{e2s}-G^{e2s}-G^{e2p}-G^{e2p}-T^{e2p}-T^{e2p}-A^{e2p}-G^{e2p}-A^{e2p}-C^{e2s}-A^{e2s}-A^{e2t}-H
Illustrative Compound No. 157: HO-T^{e2s}-A^{e2s}-G^{e2s}-G^{e2s}-G^{e2p}-T^{e2p}-T^{e2p}-A^{e2p}-G^{e2p}-A^{e2s}-C^{e2s}-A^{e2s}-A^{e2s}-CH₂CH₂OH
Illustrative Compound No. 158: HO-T^{e2s}-A^{e2s}-G^{e2s}-G^{e2s}-G^{e2p}-T^{e2p}-T^{e2p}-A^{e2p}-G^{e2p}-A^{e2s}-C^{e2s}-A^{e2s}-A^{e2t}-H
Illustrative Compound No. 159: HOP(S)(OH)-O-T^{e2s}-A^{e2s}-G^{e2s}-G^{e2s}-G^{e2s}-T^{e2s}-T^{e2s}-A^{e2s}-G^{e2s}-A^{e2s}-C^{e2s}-A^{e2s}-A^{e2s}-CH₂CH₂OH
Illustrative Compound No. 160: HOP(S)(OH)-O-T^{e2s}-A^{e2s}-G^{e2s}-G^{e2s}-G^{e2s}-T^{e2s}-T^{e2s}-A^{e2s}-G^{e2s}-A^{e2s}-C^{e2s}-A^{e2s}-A^{e2t}-H
Illustrative Compound No. 161: C₁₇H₃₅C(O)O(CH₂)₂SP(O)(OH)-O-T^{e2s}-A^{e2s}-G^{e2s}-G^{e2s}-G^{e2s}-T^{e2s}-T^{e2s}-A^{e2s}-G^{e2s}-A^{e2s}-C^{e2s}-A^{e2s}-A^{e2s}-CH₂CH₂OH
Illustrative Compound No. 162: C₁₇H₃₅C(O)O(CH₂)₂SP(O)(OH)-O-T^{e2s}-A^{e2s}-G^{e2s}-G^{e2s}-G^{e2s}-T^{e2s}-T^{e2s}-A^{e2s}-G^{e2s}-A^{e2s}-C^{e2s}-A^{e2s}-A^{e2t}-H
Illustrative Compound No. 163: C₁₈H₃₇C(O)O(CH₂)₂SP(O)(OH)-O-T^{e2s}-A^{e2s}-G^{e2s}-G^{e2s}-G^{e2s}-T^{e2s}-T^{e2s}-A^{e2s}-G^{e2s}-A^{e2s}-C^{e2s}-A^{e2s}-A^{e2s}-CH₂CH₂OH
Illustrative Compound No. 164: C₁₈H₃₇C(O)O(CH₂)₂SP(O)(OH)-O-T^{e2s}-A^{e2s}-G^{e2s}-G^{e2s}-G^{e2s}-T^{e2s}-T^{e2s}-A^{e2s}-G^{e2s}-A^{e2s}-C^{e2s}-A^{e2s}-A^{e2t}-H
Illustrative Compound No. 165: C₁₉H₃₉C(O)O(CH₂)₂SP(O)(OH)-O-T^{e2s}-A^{e2s}-G^{e2s}-G^{e2s}-G^{e2s}-T^{e2s}-T^{e2s}-A^{e2s}-G^{e2s}-A^{e2s}-C^{e2s}-A^{e2s}-A^{e2s}-CH₂CH₂OH
Illustrative Compound No. 166: C₁₉H₃₉C(O)O(CH₂)₂SP(O)(OH)-O-T^{e2s}-A^{e2s}-G^{e2s}-G^{e2s}-G^{e2s}-T^{e2s}-T^{e2s}-A^{e2s}-G^{e2s}-A^{e2s}-C^{e2s}-A^{e2s}-A^{e2t}-H
Illustrative Compound No. 167: C₁₆H₃₃C(O)O(CH₂)₂SP(O)(OH)-O-T^{e2s}-A^{e2s}-G^{e2s}-G^{e2s}-G^{e2s}-T^{e2s}-T^{e2s}-A^{e2s}-G^{e2s}-A^{e2s}-C^{e2s}-A^{e2s}-A^{e2s}-CH₂CH₂OH
Illustrative Compound No. 168: C₁₆H₃₃C(O)O(CH₂)₂SP(O)(OH)-O-T^{e2s}-A^{e2s}-G^{e2s}-G^{e2s}-G^{e2s}-T^{e2s}-T^{e2s}-A^{e2s}-G^{e2s}-A^{e2s}-C^{e2s}-A^{e2s}-A^{e2t}-H
Illustrative Compound No. 169: C₁₅H₃₁C(O)O(CH₂)₂SP(O)(OH)-O-T^{e2s}-A^{e2s}-G^{e2s}-G^{e2s}-G^{e2s}-T^{e2s}-T^{e2s}-A^{e2s}-G^{e2s}-A^{e2s}-C^{e2s}-A^{e2s}-A^{e2s}-CH₂CH₂OH
Illustrative Compound No. 170: C₁₅H₃₁C(O)O(CH₂)₂SP(O)(OH)-O-T^{e2s}-A^{e2s}-G^{e2s}-G^{e2s}-G^{e2s}-T^{e2s}-T^{e2s}-A^{e2s}-G^{e2s}-A^{e2s}-C^{e2s}-A^{e2s}-A^{e2t}-H
and a pharmacologically acceptable salt thereof.

In the above description and the present specification, G^{e1t}, C^{e1t}, A^{e1t}, T^{e1t}, A^{e1p}, G^{e1p}, C^{e1p}, T^{e1p}, A^{e1s}, G^{e1s}, C^{e1s}, T^{e1s}, G^{e2t}, C^{e2t}, A^{e2t}, T^{e2t}, A^{e2p}, G^{e2p}, C^{e2p}, T^{e2p}, A^{e2s}, G^{e2s}, C^{e2s}, and T^{e2s} represent groups having the chemical structures shown below.

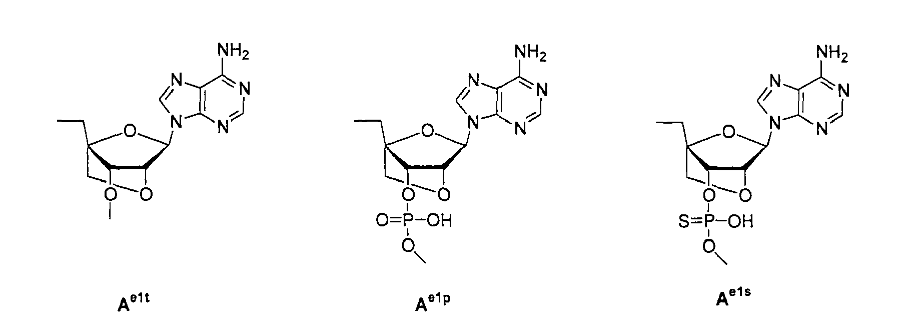

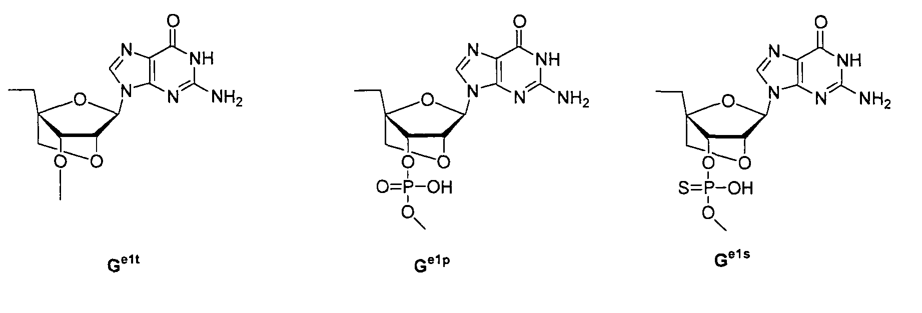

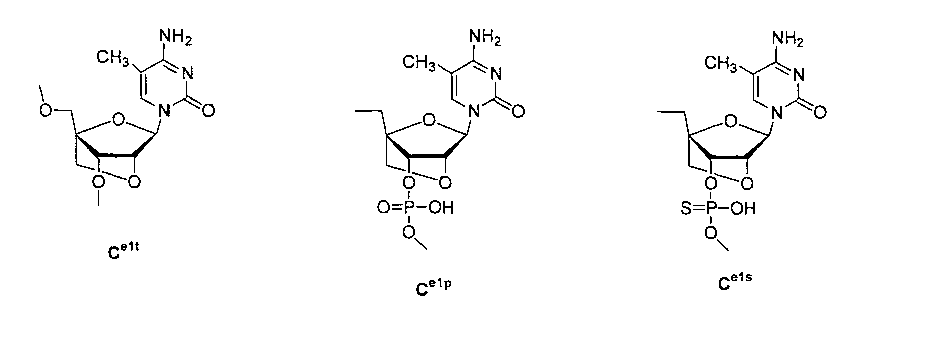

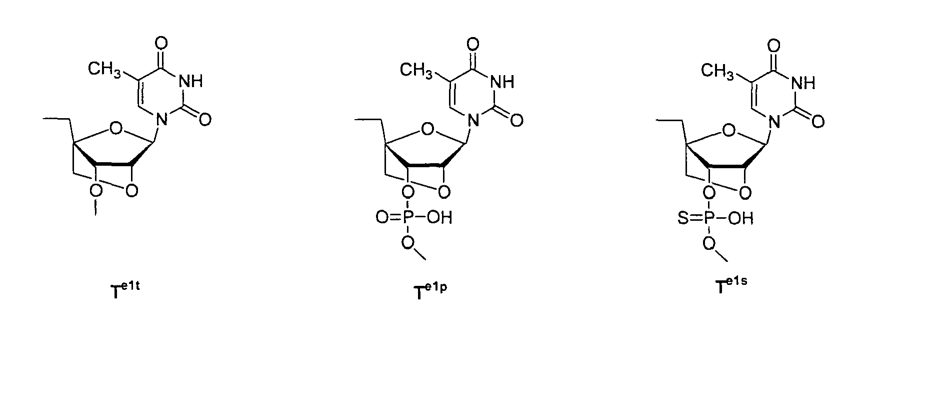

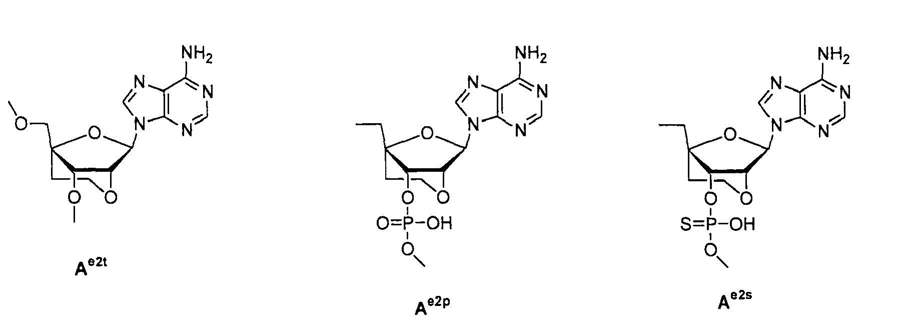

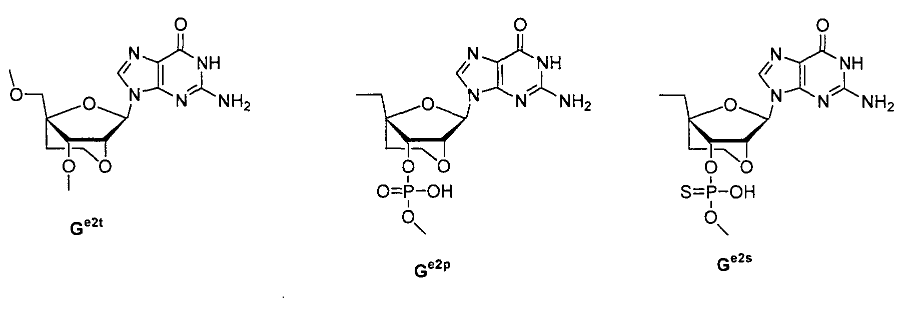

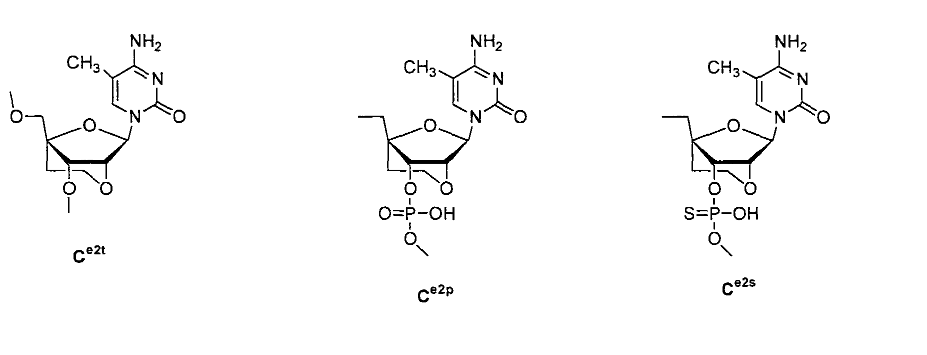

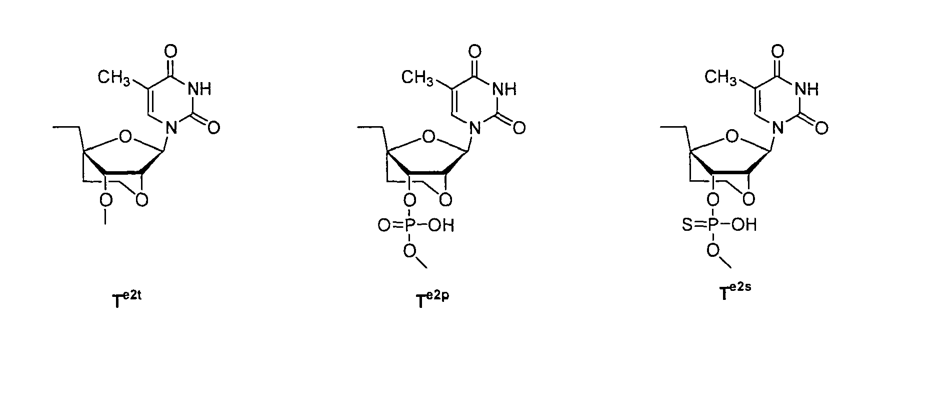

In the above-described compound group, further preferred compounds are the compounds of Exemplified Compound Nos. 1 to 13, 19 to 31, 37 to 39, 73 to 79, and 145 to 158; and still further preferred compounds are the compounds of Exemplified Compound Nos. 1 to 3, 13 to 15, 31 to 33, 43 to 45, 61 to 63, 73 to 75, 91 to 93, and 103 to 105.

"Pharmacologically acceptable salt thereof' refers to a salt of a compound of the present invention because the compound can be made in the form of a salt, and preferred examples of the salt can include alkali metal salts such as sodium, potassium, and lithium; alkali earth metal salts such as calcium and magnesium; metal salts such as aluminum, iron, zinc, copper, nickel, and cobalt; amine salts such as inorganic salts (e.g., ammonium salts) and organic salts (e.g., t-octylamine, dibenzylamine, morpholine, glucosamine, phenylglycine alkyl ester, ethylenediamine, N-methylglucamine, guanidine, diethylamine, triethylamine, dicyclohexylamine, N,N'-dibenzylethylenediamine, chloroprocaine, procaine, diethanolamine, N-benzyl-phenethylamine, piperazine, tetramethylammonium and tris(hydroxymethyl)aminomethane salts); inorganic acid salts such as hydrohalides (e.g., hydrofluorides, hydrochlorides, hydrobromides, and hydroiodides), nitrates, perchlorates, sulfates, and phosphates; organic acid salts such as lower alkanesulfonates (e.g., methanesulfonates, trifluoromethanesulfonates, and ethanesulfonates), arylsulfonates (e.g., benzenesulfonates and p-toluenesulfonates), acetates, malates, fumarates, succinates, citrates, tartrates, oxalates, and maleates; and amino-acid salts such as glycine salts, lysine salts, arginine salts, ornithine salts, glutamates, and aspartates.

The compound (I) of the present invention may be present in the form of a hydrate, and the invention also includes a hydrate thereof.

Another aspect of the present invention is a medicine containing the compound (I) of the present invention, preferably a medicine used for treating or preventing diseases in which telomerase is involved, more preferably a medicine used for treating or preventing cancer.

The compound of the present invention strongly binds to telomerase RNA, and is useful for treating or preventing diseases in which telomerase is involved, such as cancer.

### Brief Description of the Drawings

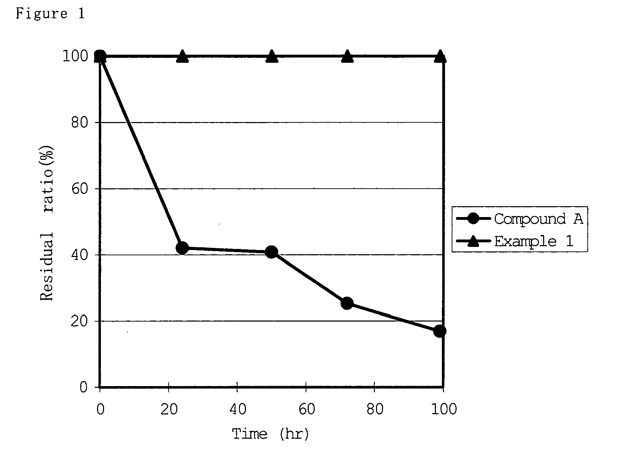

Figure 1 shows the results of a stability test in Test Example 2. Time: the time elapsed from the start of the test. Residual amount: the residual amount when it is 100% at the time of the start.

### Best Mode for Carrying Out the Invention

The compound (I) of the present invention can be synthesized according to the method described in a reference (Nucleic Acids Research, 12: 4539 (1984)) using a DNA synthesizer, for example, Perkin Elmer's Model 392 employing a phosphoroamidite method.

For natural nucleosides, the phosphoroamidite reagents used in this instance may be commercially available ones; for other nucleosides, they may be obtained according to the method described in WO99/14226 when 1 to w are each 1 therein or according to that in WO00/47599 when 1 to w are each 2 to 5 therein.

In addition, the compound (I) may be optionally thioated according to a method described in a reference. (Tetrahedron Letters, 32: 3005 (1991), J. Am. Chem. Soc., 112: 1253 (1990)) using a reagent reacting with trivalent phosphorus to form a thioether, for example, tetraethylthiuram disulfide (TETD, Applied Biosystems), xanthan hydride, or Beaucage reagent (Glen Research) to provide a thioate derivative thereof.

The compound (I) having 3,4-DBB can be synthesized by a method described in Japanese Patent Laid-Open No. 07-87892 or 11-199597. The compound (I) having an acyloxyethyl thiophosphate group can be synthesized by a method described in Japanese Patent Laid-Open No. 2004-182725.

The compound (I) of the present invention or a pharmacologically acceptable salt thereof has the activity of inhibiting telomerase. In addition, the compound (I) of the present invention is excellent in its pharmacokinetics such as absorption, biodistribution, and half-life in the blood, and also has low toxicity to organs such as the kidney and liver. Thus, the compound (I) of the present invention is useful, for example, as a medicine, particularly for treating or preventing diseases in which various telomerases are involved (cancer in particular).

When the compound of the present invention is used as an agent for preventing or treating the above-mentioned diseases, the compound of the above-described general formula (I) or a pharmacologically acceptable salt thereof may be administered alone or in a suitable mixture with a pharmacologically acceptable excipient, diluent and the like orally in the form of, for example, a tablet, capsule, granule, powder, or syrup or parenterally in the form of, for example, an injection, suppository, patch, or external preparation.

These preparations may be produced by well-known methods using additives such as excipients (including, for example, organic excipients such as sugar derivatives (e.g., lactose, saccharose, glucose, mannitol, and sorbitol), starch derivatives (e.g., corn starch, potato starch, α-starch, and dextrin), cellulose derivatives (e.g., crystalline cellulose), gum arabic, dextran, and pullulan and inorganic excipients such as silicate derivatives (e.g., light silicic acid anhydride, synthetic aluminium silicate, calcium silicate, and magnesium metasilicate aluminate), phosphates (e.g., calcium hydrogen phosphate), carbonates (e.g., calcium carbonate), and sulfates (e.g., calcium sulfate)), lubricants (including, for example, stearic acid or its metal salts (e.g., calcium stearate and magnesium stearate), talc, colloidal silica, waxes (e.g., bees wax and spermaceti), boric acid, adipic acid, sulfates (e.g., sodium sulfate), glycol, fumaric acid, sodium benzoate, DL-leucine, lauryl sulfates (e.g., sodium lauryl sulfate and magnesium lauryl sulfate), silicic acids (e.g., silicic acid anhydride and silicic acid hydrate), and the above-described starch derivatives), binders (including, for example, hydroxypropylcellulose, hydroxypropyl methylcellulose, polyvinylpyrrolidone, macrogol, and compounds similar to the above-described excipients), disintegrators (including, for example, cellulose derivatives (e.g., low substituted hydroxypropylcellulose, carboxymethylcellulose, carboxymethylcellulose calcium, and internally cross-linked carboxymethylcellulose sodium) and chemically modified starches or celluloses (e.g., carboxymethylstarch, carboxymethylstarch sodium, and cross-linked polyvinylpyrrolidone)), emulsifiers (including, for example, colloidal clays (e.g., bentonite and veegum), metal hydroxides (e.g., magnesium hydroxide and aluminium hydroxide), anionic surface active agents (e.g., sodium lauryl sulfate and calcium stearate), cationic surface active agents (e.g., benzalkonium chloride), and nonionic surfactants (e.g., polyoxyethylene alkyl ether, polyoxyethylene sorbitan fatty acid ester, and sucrose fatty acid ester)), stabilizers (including, for example, p-hydroxybenzoic esters (e.g., methylparaben and propylparaben), alcohols (chlorobutanol, benzyl alcohol, and phenylethyl alcohol), benzalkonium chloride, phenols (e.g., phenol and cresol), thimerosal, dehydroacetic acid, and sorbic acid), flavoring agents (including, for example, conventionally used sweeteners, acidifiers and perfumes), and diluents.

In addition to the above-described additives, a colloidal dispersion system may be used in a method for introducing the compound of the present invention to patients. The colloidal dispersion system is expected to have the effect of enhancing the stability of a compound in the body and the effect of efficiently transporting the compound to a particular organ, tissue or cell. The colloidal dispersion system is not restricted if it is a conventionally used one; examples thereof include lipid-based dispersion systems including a macromolecular complex, nanocapsule, microsphere, bead, oil-in-water emulsifier, micelle, mixed micelle, and liposome. A preferred colloidal dispersion system is a plurality of liposomes or artificial membrane vesicles which have the effect of efficiently transporting a compound to a particular organ, tissue or cell (Mannino, et al., Biotechniques, 1988, 6: 682; Blume and Cevc, Biochem. et Biophys. Acta, 1990, 1029: 91; Lappalainen, et al., Antiviral Res., 1994, 23: 119; Chonn and Cullis, Current Op. Biotech., 1995, 6: 698).

Unilamellar liposomes having a size range of 0.2 to 0.4 µm can encapsulate a substantial percentage of an aqueous buffer containing large macromolecules, and a compound is encapsulated within the aqueous interior and delivered to brain cells in a biologically active form (Fraley, et al., Trends Biochem. Sci., 1981, 6: 77). The composition of the liposome is usually a combination of lipids, particularly phospholipids, in particular, high phase transition temperature phospholipids, usually with one or more steroids, particularly cholesterol. Examples of lipids useful in liposome production include phosphatidyl compounds, such as phosphatidylglycerol, phosphatidylcholine, phosphatidylserine, sphingolipids, phosphatidylethanolamine, cerebrosides, and gangliosides. Particularly useful are diacyl phosphatidylglycerols, where the lipid moiety contains 14-18 carbon atoms, particularly 16-18 carbon atoms, and is saturated (lacking double bonds within the 14-18 carbon atom chain). Typical phospholipids include phosphatidylcholine, dipalmitoylphosphatidylcholine, and distearoylphosphatidylcholine.

The targeting of colloidal dispersion systems, including liposomes, can be either passive or active. Passive targeting is achieved by utilizing the natural tendency of liposomes to distribute to cells of the reticuloendothelial system in organs that contain sinusoidal capillaries. Active targeting, by contrast, involves modification of the liposome by coupling thereto a specific ligand such as a viral protein coat (Morishita et al., Proc. Natl. Acad. Sci. (U.S.A.), 1993, 90: 8474), monoclonal antibody (or a suitable binding portion thereof), sugar, glycolipid or protein (or a suitable oligopeptide fragment thereof), or by changing the composition of the liposome in order to achieve distribution to organs and cell types other than the naturally occurring sites of localization. The surface of the targeted colloidal dispersion system can be modified in a variety of ways. In the case of a liposomal targeted delivery system, lipid groups can be incorporated into the lipid bilayer of the liposome in order to maintain the targeting ligand in close association with the lipid bilayer. Various linking groups can be used for joining the lipid chains to the targeting ligand. The targeting ligand, which binds a specific cell surface molecule found predominantly on cells to which delivery of the oligonucleotides of the present invention is desired, may be, for example, (1) a hormone, growth factor or a suitable oligopeptide fragment thereof which is bound by a specific cellular receptor predominantly expressed by cells to which delivery is desired or (2) a polyclonal or monoclonal antibody, or a suitable fragment thereof (e.g., Fab; F(ab')2) which specifically binds an antigenic epitope found predominantly on targeted cells. Two or more bioactive agents (e.g., the compound (I) and other agents) can be combined within, and delivered by, a single liposome. It is also possible to add agents to colloidal dispersion systems which enhance the intercellular stability and/or targeting of the contents thereof.

The usage amount of the compound (I) of the present invention or a pharmacologically acceptable salt thereof will vary depending on symptoms, age, and the like, but is desirably administered to an adult once to six times a day orally at a lower limit of 1 mg (preferably 30 mg) per once and an upper limit of 2,000 mg (preferably 1,500 mg) per once or intravenously at a lower limit of 0.5 mg (preferably 5 mg) per once and an upper limit of 500 mg (preferably 250 mg) per once, depending on the symptoms.

The present invention is described below in further detail with reference to Examples, Reference Example, and Test Examples.

### Example 1

Synthesis of HO-T^{e2s}-A^{e2s}-G^{e2s}-G^{e2s}-G^{e2s}-T^{e2s}-T^{e2s}-A^{e2s}-G^{e2s}-A^{e2s}-C^{e2s}-A^{e2s}-A^{e2s}-CH₂CH₂OH (Exemplified Compound No. 1)

The synthesis was carried out at a scale of 1 µmol using an automated nucleic acid synthesizer (ABI Model 394 DNA/RNA Synthesizer from Perkin Elmer). The concentrations of the solvent, reagent, and phosphoramidite in each synthesis cycle were the same as those for the synthesis of natural oligonucleotides. For sulfuration was used 0.3% xanthan hydride/pyridine:acetonitrile = 1:9 (v/v) (900 seconds), and other solvents and reagents were obtained from Applied Biosystems. The non-natural phosphoramidites used were compounds described in Example 14 (5'-O-dimethoxytrityl-2'-O,4'-C-ethylene-6-N-benzoyladenosine-3'-O-(2-cyanoethyl-N,N-diisopropyl)phosphoramidite), Example 27 (5'-O-dimethoxytrityl-2'-O,4'-C-ethylene-2-N-isobutylylguanosine-3'-O-(2-cyanoethyl-N,N-diisopropyl)phosphoramidite), Example 22 (5'-O-dimethoxytrityl-2'-O,4'-C-ethylene-4-N-benzoyl-5-methylcytidine-3'-O-(2-cyanoethyl-N,N-diisopropyl)phosphoramidite) and Example 9 (5'-O-dimethoxytrityl-2'-O,4'-C-ethylene-5-methyluridine-3'-O-(2-cyanoethyl-N,N-diisopropyl)phosphoramidite) of Japanese Patent Laid-Open No. 2000-297097. A controlled pore glass (CPG) modified by dimethoxytritylethylene glycol (described in Example 12b of Japanese Patent Laid-Open No. 07-87982) was used in an amount of 1.2 µmol as a solid support to synthesize the title compound.

A protected oligonucleotide analogue having a desired sequence was treated with concentrated ammonia water to cleave the oligomer from the support and simultaneously to remove protective cyanoethyl groups on the phosphorus atoms and protective groups on the nucleic acid bases. The solvent was distilled off under reduced pressure, followed by purifying the resultant residue using reverse phase HPLC (LC-10VP from Shimadzu Corporation, column (Merck, Chromolith Performance RP-18e (4.6×100 mm)), A solution: 5% acetonitrile, 0.1 M triethylamine acetate aqueous solution (TEAA), pH 7.0, B solution: acetonitrile, B%: 20%→60% (10 min., linear gradient); 60°C; 2 ml/min.; 254 nm) to collect the peak of the desired compound having dimethoxytrityl groups. Water was added, followed by distilling off under reduced pressure to remove TEAA. An 80% acetic acid aqueous solution (200 µl) was added to the residue, followed by allowing to stand for 20 minutes to deprotect the dimethoxytrityl groups. The solvent was distilled off, followed by purification using ion exchange HPLC (LC-10VP from Shimadzu Corporation, column (Tosoh Corporation, DEAE-5PW (10×50 mm)), A solution: 20% acetonitrile aqueous solution, B solution: 20% acetonitrile, 67 mM phosphate buffer, 1.5 M potassium bromide aqueous solution, pH 6.8 B%: 20%→70% (10 min., linear gradient); 60°C; 2 ml/min.; 254 nm) to collect the peak of the desired compound. The solvent was distilled off under reduced pressure, followed by dissolving the residue in 2 ml of water, and desalting by gel filtration using Sephadex G-25 (15×300 mm). A fraction corresponding to the desired compound was collected and distilled off, followed by dissolving the residue in 1 ml of water before filtration using a 0.45-µm filter (Millipore, Ultrafree-MC) to provide the desired oligonucleotide. When analyzed using ion exchange HPLC (LC-10VP from Shimadzu Corporation, column (Tosoh Corporation, DEAE-5PW (10×50 mm)), A solution: 20% acetonitrile aqueous solution, B solution: 20% acetonitrile, 67 mM phosphate buffer, 1.5 M potassium bromide aqueous solution, pH 6.8 B%: 20%→70% (10 min., linear gradient); 60°C; 2 ml/min.; 254 nm), this compound was eluted at 6.39 minutes (26.4 A₂₆₀ units) (λₘₐₓ (H₂O) = 257 nm). In addition, the compound was identified by negative ion ESI mass spectrometry (calculated value: 4916.10, measured value: 4916.09).

The base sequence of the compound is a sequence complementary to the nucleotide Nos. 136-148 region of human telomerase RNA (GenBank accession No. U86046).

### Example 2

Synthesis of HO-T^{e2s}-A^{e2s}-G^{e2s}-G^{e2s}-G^{e2s}-T^{e2s}-T^{e2s}-A^{e2s}-G^{e2s}-A^{e2s}-C^{e2s}-A^{e2s}-CH₂CH₂OH (Exemplified Compound No. 2)

The compound of Example 2 having the desired sequence was synthesized in the same manner as the compound of Example 1 using 1.2 µmol of CPG identical to that in Example 1. After deprotection, the purification was carried out using reverse phase HPLC (LC-10VP from Shimadzu Corporation, column (Merck, Chromolith Performance RP-18e (4.6×100 mm)), A solution: 5% acetonitrile, 0.1 M triethylamine acetate aqueous solution (TEAA), pH 7.0, B solution: acetonitrile, B%: 10%→50% (10 min., linear gradient); 60°C; 2 ml/min.; 254 nm) to collect the peak of the desired compound having dimethoxytrityl groups.

The dimethoxytrityl groups were deprotected, followed by purification using ion exchange HPLC (LC-10VP from Shimadzu Corporation, column (Tosoh Corporation, DEAE-5PW (10×50 mm)), A solution: 20% acetonitrile aqueous solution, B solution: 20% acetonitrile, 67 mM phosphate buffer, 1.5 M potassium bromide aqueous solution, pH 6.8 B%: 20%→70% (10 min., linear gradient); 60°C; 2 ml/min.; 254 nm) to collect the peak of the desired compound. The desalting was carried out by gel filtration using Sephadex G-25 (15×300 mm). When analyzed using ion exchange HPLC (LC-10VP from Shimadzu Corporation, column (Tosoh Corporation, DEAE-5PW (10×50 mm)), A solution: 20% acetonitrile aqueous solution, B solution: 20% acetonitrile, 67 mM phosphate buffer, 1.5 M potassium bromide aqueous solution, pH 6.8 B%: 20%→70% (10 min., linear gradient); 60°C; 2 ml/min.; 254 nm), the finally purified compound was eluted at 5.73 minutes (11.5 A₂₆₀ units) (λₘₐₓ (H₂O) = 259 nm). In addition, the compound was identified by negative ion ESI mass spectrometry (calculated value: 4544.79, measured value: 4543.92).

The base sequence of the compound is a sequence complementary to the nucleotide Nos. 137-148 region of human telomerase RNA (GenBank accession No. U86046).

### Example 3

### Synthesis of HO-T^{e2s}-A^{e2s}-G^{e2s}-G^{e2s}-G^{e2s}-T^{e2s}-T^{e2s}-A^{e2s}-G^{e2s}-A^{e2s}-CH₂CH₂OH (Exemplified Compound No. 4)

The compound of Example 3 having the desired sequence was synthesized in the same manner as the compound of Example 1 using 1.2 µmol of CPG identical to that in Example 1. After deprotection, the purification was carried out using reverse phase HPLC (LC-10VP from Shimadzu Corporation, column (Merck, Chromolith Performance RP-18e (4.6×100 mm)), A solution: 5% acetonitrile, 0.1 M triethylamine acetate aqueous solution (TEAA), pH 7.0, B solution: acetonitrile, B%: 10%→50% (10 min., linear gradient); 60°C; 2 ml/min.; 254 nm) to collect the peak of the desired compound having dimethoxytrityl groups.

The dimethoxytrityl groups were deprotected, followed by purification using ion exchange HPLC (LC-10VP from Shimadzu Corporation, column (Tosoh Corporation, DEAE-5PW (10×50 mm)), A solution: 20% acetonitrile aqueous solution, B solution: 20% acetonitrile, 67 mM phosphate buffer, 1.5 M potassium bromide aqueous solution, pH 6.8 B%: 20%→70% (10 min., linear gradient); 60°C; 2 ml/min.; 254 nm) to collect the peak of the desired compound. The desalting was carried out by gel filtration using Sephadex G-25 (15×300 mm). When analyzed using ion exchange HPLC (LC-10VP from Shimadzu Corporation, column (Tosoh Corporation, DEAE-5PW (10×50 mm)), A solution: 20% acetonitrile aqueous solution, B solution: 20% acetonitrile, 67 mM phosphate buffer, 1.5 M potassium bromide aqueous solution, pH 6.8 B%: 20%→70% (10 min., linear gradient); 60°C; 2 ml/min.; 254 nm), the finally purified compound was eluted at 4.54 minutes (6.88 A₂₆₀ units) (λₘₐₓ (H₂O) = 254 nm). In addition, the compound was identified by negative ion ESI mass spectrometry (calculated value: 3812.16, measured value: 3811.17).

The base sequence of the compound is a sequence complementary to the nucleotide Nos. 139-148 region of human telomerase RNA (GenBank accession No. U86046).

### Example 4

### Synthesis of HO-T^{e2s}-A^{e2s}-G^{e2s}-G^{e2s}-G^{e2s}-T^{e2s}-T^{e2s}-A^{e2s}-CH₂CH₂OH (Exemplified Compound No. 6)

The compound of Example 4 having the desired sequence was synthesized in the same manner as the compound of Example 1 using 1.2 µmol of CPG identical to that in Example 1. After deprotection, the purification was carried out using reverse phase HPLC (LC-10VP from Shimadzu Corporation, column (Merck, Chromolith Performance RP-18e (4.6×100 mm)), A solution: 5% acetonitrile, 0.1 M triethylamine acetate aqueous solution (TEAA), pH 7.0, B solution: acetonitrile, B%: 10%→50% (10 min., linear gradient); 60°C; 2 ml/min.; 254 nm) to collect the peak of the desired compound having dimethoxytrityl groups.

The dimethoxytrityl groups were deprotected, followed by purification using ion exchange HPLC (LC-10VP from Shimadzu Corporation, column (Tosoh Corporation, DEAE-5PW (10×50 mm)), A solution: 20% acetonitrile aqueous solution, B solution: 20% acetonitrile, 67 mM phosphate buffer, 1.5 M potassium bromide aqueous solution, pH 6.8 B%: 20%→70% (10 min., linear gradient); 60°C; 2 ml/min.; 254 nm) to collect the peak of the desired compound. The desalting was carried out by gel filtration using Sephadex G-25 (15×300 mm). When analyzed using ion exchange HPLC (LC-10VP from Shimadzu Corporation, column (Tosoh Corporation, DEAE-5PW (10×50 mm)), A solution: 20% acetonitrile aqueous solution, B solution: 20% acetonitrile, 67 mM phosphate buffer, 1.5 M potassium bromide aqueous solution, pH 6.8 B%: 20%→70% (10 min., linear gradient); 60°C; 2 ml/min.; 254 nm), the finally purified compound was eluted at 5.53 minutes (8.73 A₂₆₀ units) (λₘₐₓ (H₂O) = 260 nm). In addition, the compound was identified by negative ion ESI mass spectrometry (calculated value: 3053.53, measured value: 3053.09).

The base sequence of the compound is a sequence complementary to the nucleotide Nos. 141-148 region of human telomerase RNA (GenBank accession No. U86046).

### Example 5

### Synthesis of HO-T^{e2s}-A^{e2s}-G^{e2s}-G^{e2s}-G^{e2s}-T^{e2s}-T^{e2s}-CH₂CH₂OH (Exemplified Compound No. 7)

The compound of Example 5 having the desired sequence was synthesized in the same manner as the compound of Example 1 using 1.2 µmol of CPG identical to that in Example 1. After deprotection, the purification was carried out using reverse phase HPLC (LC-10VP from Shimadzu Corporation, column (Merck, Chromolith Performance RP-18e (4.6×100 mm)), A solution: 5% acetonitrile, 0.1 M triethylamine acetate aqueous solution (TEAA), pH 7.0, B solution: acetonitrile, B%: 10%→50% (10 min., linear gradient); 60°C; 2 ml/min.; 254 nm) to collect the peak of the desired compound having dimethoxytrityl groups.

The dimethoxytrityl groups were deprotected, followed by purification using ion exchange HPLC (LC-10VP from Shimadzu Corporation, column (Tosoh Corporation, DEAE-5PW (10×50 mm)), A solution: 20% acetonitrile aqueous solution, B solution: 20% acetonitrile, 67 mM phosphate buffer, 1.5 M potassium bromide aqueous solution, pH 6.8 B%: 20%→70% (10 min., linear gradient); 60°C; 2 ml/min.; 254 nm) to collect the peak of the desired compound. The desalting was carried out by gel filtration using Sephadex G-25 (15×300 mm). When analyzed using ion exchange HPLC (LC-10VP from Shimadzu Corporation, column (Tosoh Corporation, DEAE-5PW (10×50 mm)), A solution: 20% acetonitrile aqueous solution, B solution: 20% acetonitrile, 67 mM phosphate buffer, 1.5 M potassium bromide aqueous solution, pH 6.8 B%: 20%→70% (10 min., linear gradient); 60°C; 2 ml/min.; 254 nm), the finally purified compound was eluted at 7.10 minutes (8.92 A₂₆₀ units) (λₘₐₓ (H₂O) = 255 nm). In addition, the compound was identified by negative ion ESI mass spectrometry (calculated value: 2682.22, measured value: 2682.10).

The base sequence of the compound is a sequence complementary to the nucleotide Nos. 142-148 region of human telomerase RNA (GenBank accession No. U86046).

### Example 6

### Synthesis of HO-T^{e2s}-A^{e2s}-G^{e2s}-G^{e2s}-G^{e2s}-T^{e2s}-T^{e2s}-A^{e2s}-G^{e2s}-CH₂CH₂OH (Exemplified Compound No. 5)

The compound of Example 6 having the desired sequence was synthesized in the same manner as the compound of Example 1 using 1.2 µmol of CPG identical to that in Example 1. After deprotection, the purification was carried out using reverse phase HPLC (LC-10VP from Shimadzu Corporation, column (Merck, Chromolith Performance RP-18e (4.6×100 mm)), A solution: 5% acetonitrile, 0.1 M triethylamine acetate aqueous solution (TEAA), pH 7.0, B solution: acetonitrile, B%: 10%→50% (10 min., linear gradient); 60°C; 2 ml/min.; 254 nm) to collect the peak of the desired compound having dimethoxytrityl groups.

The dimethoxytrityl groups were deprotected, followed by purification using ion exchange HPLC (LC-10VP from Shimadzu Corporation, column (Tosoh Corporation, DEAE-5PW (10×50 mm)), A solution: 20% acetonitrile aqueous solution, B solution: 20% acetonitrile, 67 mM phosphate buffer, 1.5 M potassium bromide aqueous solution, pH 6.8 B%: 20%→70% (10 min., linear gradient); 60°C; 2 ml/min.; 254 nm) to collect the peak of the desired compound. The desalting was carried out by gel filtration using Sephadex G-25 (15×300 mm). When analyzed using ion exchange HPLC (LC-10VP from Shimadzu Corporation, column (Tosoh Corporation, DEAE-5PW (10×50 mm)), A solution: 20% acetonitrile aqueous solution, B solution: 20% acetonitrile, 67 mM phosphate buffer, 1.5 M potassium bromide aqueous solution, pH 6.8 B%: 20%→70% (10 min., linear gradient); 60°C; 2 ml/min.; 254 nm), the finally purified compound was eluted at 7.67 minutes (19.5 A₂₆₀ units) (λₘₐₓ (H₂O) = 259 nm). In addition, the compound was identified by negative ion ESI mass spectrometry (calculated value: 3440.85, measured value: 3439.47).

The base sequence of the compound is a sequence complementary to the nucleotide Nos. 140-148 region of human telomerase RNA (GenBank accession No. U86046).

### Example 7

### Synthesis of HO-A^{e2s}-G^{e2s}-G^{e2s}-G^{e2s}-T^{e2s}-T^{e2s}-A^{e2s}-G^{e2s}-A^{e2s}-CH₂CH₂OH (Exemplified Compound No. 11)

The compound of Example 7 having the desired sequence was synthesized in the same manner as the compound of Example 1 using 1.2 µmol of CPG identical to that in Example 1. After deprotection, the purification was carried out using reverse phase HPLC (LC-10VP from Shimadzu Corporation, column (Merck, Chromolith Performance RP-18e (4.6×100 mm)), A solution: 5% acetonitrile, 0.1 M triethylamine acetate aqueous solution (TEAA), pH 7.0, B solution: acetonitrile, B%: 10%→50% (10 min., linear gradient); 60°C; 2 ml/min.; 254 nm) to collect the peak of the desired compound having dimethoxytrityl groups.

The dimethoxytrityl groups were deprotected, followed by purification using ion exchange HPLC (LC-10VP from Shimadzu Corporation, column (Tosoh Corporation, DEAE-5PW (10×50 mm)), A solution: 20% acetonitrile aqueous solution, B solution: 20% acetonitrile, 67 mM phosphate buffer, 1.5 M potassium bromide aqueous solution, pH 6.8 B%: 20%→70% (10 min., linear gradient); 60°C; 2 ml/min.; 254 nm) to collect the peak of the desired compound. The desalting was carried out by gel filtration using Sephadex G-25 (15×300 mm). When analyzed using ion exchange HPLC (LC-10VP from Shimadzu Corporation, column (Tosoh Corporation, DEAE-5PW (10×50 mm)), A solution: 20% acetonitrile aqueous solution, B solution: 20% acetonitrile, 67 mM phosphate buffer, 1.5 M potassium bromide aqueous solution, pH 6.8 B%: 20%→70% (10 min., linear gradient); 60°C; 2 ml/min.; 254 nm), the finally purified compound was eluted at 7.60 minutes (18.6 A₂₆₀ units) (λₘₐₓ (H₂O) = 257 nm). In addition, the compound was identified by negative ion ESI mass spectrometry (calculated value: 3449.86, measured value: 3449.42).

The base sequence of the compound is a sequence complementary to the nucleotide Nos. 139-147 region of human telomerase RNA (GenBank accession No. U86046).

### Example 8

### Synthesis of HO-T^{e2s}-A^{e2s}-G^{e2s}-G^{e2s}-G^{e2s}-T^{e2s}-T^{e2s}-A^{e2s}-G^{e2s}-A^{e2s}-C^{e2s}-A^{e2s}-A^{e2t}-H (Exemplified Compound No. 19)

The compound of Example 8 having the desired sequence was synthesized in the same manner as the compound of Example 1 using 1.2 µmol of universal-Q 500 CPG (from Glen Research). After deprotection, the purification was carried out using reverse phase HPLC (LC-10VP from Shimadzu Corporation, column (Merck, Chromolith Performance RP-18e (4.6×100 mm)), A solution: 5% acetonitrile, 0.1 M triethylamine acetate aqueous solution (TEAA), pH 7.0, B solution: acetonitrile, B%: 10%→50% (10 min., linear gradient); 60°C; 2 ml/min.; 254 nm) to collect the peak of the desired compound having dimethoxytrityl groups.

The dimethoxytrityl groups were deprotected, followed by purification using ion exchange HPLC (LC-10VP from Shimadzu Corporation, column (Tosoh Corporation, DEAE-5PW (10×50 mm)), A solution: 20% acetonitrile aqueous solution, B solution: 20% acetonitrile, 67 mM phosphate buffer, 1.5 M potassium bromide aqueous solution, pH 6.8 B%: 20%→70% (10 min., linear gradient); 60°C; 2 ml/min.; 254 nm) to collect the peak of the desired compound. The desalting was carried out by gel filtration using Sephadex G-25 (15×300 mm). When analyzed using ion exchange HPLC (LC-10VP from Shimadzu Corporation, column (Tosoh Corporation, DEAE-5PW (10×50 mm)), A solution: 20% acetonitrile aqueous solution, B solution: 20% acetonitrile, 67 mM phosphate buffer, 1.5 M potassium bromide aqueous solution, pH 6.8 B%: 20%→70% (10 min., linear gradient); 60°C; 2 ml/min.; 254 nm), the finally purified compound was eluted at 9.55 minutes (18.6 A₂₆₀ units) (λₘₐₓ (H₂O) = 264 nm). In addition, the compound was identified by negative ion ESI mass spectrometry (calculated value: 4776.00, measured value: 4775.23).

The base sequence of the compound is a sequence complementary to the nucleotide Nos. 136-148 region of human telomerase RNA (GenBank accession No. U86046).

### Example 9

### Synthesis of 3,4-DBB-(CH₂)₆-O-P(=S)(OH)-O-T^{e2s}-A^{e2s}-G^{e2s}-G^{e2s}-G^{e2s}-T^{e2s}-T^{e2s}-A^{e2s}-G^{e2s}-A^{e2s}-C^{e2s}-A^{e2s}-A^{e2s}-CH₂CH₂OH (Exemplified Compound No. 147)

The compound of Example 9 having the desired sequence was synthesized in the same manner as the compound of Example 1 using 2.7 µmol of CPG identical to that in Example 1. Described in Example 13b of Japanese Patent Laid-Open No. 11-199597, 6-O-[(3,4-dibenzyloxy)benzyl]-hexanediol-1-O-(2-cyanoethyl N,N-diisopropyl)phosphoramidite was lastly coupled. After deprotection, the purification was carried out using reverse phase HPLC (LC-10VP from Shimadzu Corporation, column (Merck, Chromolith Performance RP-18e (4.6×100 mm)), A solution: 5% acetonitrile, 0.1 M triethylamine acetate aqueous solution (TEAA), pH 7.0, B solution: acetonitrile, B%: 10%→50% (8 min., linear gradient); 60°C; 2 ml/min.; 254 nm) to collect the peak of the desired compound having a 3,4-DBB group.

When analyzed using reverse phase HPLC (LC-10VP from Shimadzu Corporation, column (Merck, Chromolith Performance RP-18e (4.6×100 mm)), A solution: 5% acetonitrile, 0.1 M triethylamine acetate aqueous solution (TEAA), pH 7.0, B solution: acetonitrile, B%: 10%→50% (8 min., linear gradient); 60°C; 2 ml/min.; 254 nm), the finally purified compound was eluted at 8.33 minutes (34.8 A₂₆₀ units) (λₘₐₓ (H₂O) = 258 nm). In addition, the compound was identified by negative ion ESI mass spectrometry (calculated value: 5414.69, measured value: 5414.48).

The base sequence of the compound is a sequence complementary to the nucleotide Nos. 137-148 region of human telomerase RNA (GenBank accession No. U86046).

### Example 10

### Synthesis of 3,4-DBB-(CH₂)₃-O-P(=S)(OH)-O-T^{e2s}-A^{e2s}-G^{e2s}-G^{e2s}-G^{e2s}-T^{e2s}-T^{e2s}-A^{e2s}-G^{e2s}-A^{e2s}-C^{e2s}-A^{e2s}-A^{e2s}-CH₂CH₂OH (Exemplified Compound No. 145)

The compound of Example 10 having the desired sequence was synthesized in the same manner as the compound of Example 1 using 2.7 µmol of CPG identical to that in Example 1. Described in Example 12b of Japanese Patent Laid-Open No. 11-199597, 6-O-[(3,4-dibenzyloxy)benzyl]-propanediol-1-O-(2-cyanoethyl N,N-diisopropyl)phosphoramidite was lastly coupled. After deprotection, the purification was carried out using reverse phase HPLC (LC-10VP from Shimadzu Corporation, column (Merck, Chromolith Performance RP-18e (4.6×100 mm)), A solution: 5% acetonitrile, 0.1 M triethylamine acetate aqueous solution (TEAA), pH 7.0, B solution: acetonitrile, B%: 10%→50% (8 min., linear gradient); 60°C; 2 ml/min.; 254 nm) to collect the peak of the desired compound having a 3,4-DBB group.

When analyzed using reverse phase HPLC (LC-10VP from Shimadzu Corporation, column (Merck, Chromolith Performance RP-18e (4.6×100 mm)), A solution: 5% acetonitrile, 0.1 M triethylamine acetate aqueous solution (TEAA), pH 7.0, B solution: acetonitrile, B%: 10%→50% (8 min., linear gradient); 60°C; 2 ml/min.; 254 nm), the finally purified compound was eluted at 7.52 minutes (28.4 A₂₆₀ units) (λₘₐₓ (H₂O) = 258 nm). In addition, the compound was identified by negative ion ESI mass spectrometry (calculated value: 5372.61, measured value: 5372.40).

The base sequence of the compound is a sequence complementary to the nucleotide Nos. 137-148 region of human telomerase RNA (GenBank accession No. U86046).

### Example 11

### Synthesis of 3,4-DBB-T^{s}-A^{e2s}-G^{e2s}-G^{e2s}-G^{e2s}-T^{e2s}-T^{e2s}-A^{e2s}-G^{e2s}-A^{e2s}-C^{e2s}-A^{e2s}-A^{e2s}-CH₂CH₂OH (Exemplified Compound No. 151)

The compound of Example 11 having the desired sequence was synthesized in the same manner as the compound of Example 1 using 2.7 µmol of CPG identical to that in Example 1. Described in Example 12b of Japanese Patent Laid-Open No. 07-87982, 5'-O-[(3,4-dibenzyloxy)benzyl]-thymidine-3'-O-(2-cyanoethyl N,N-diisopropyl)phosphoramidite was lastly coupled thereto. After deprotection, the purification was carried out using reverse phase HPLC (LC-10VP from Shimadzu Corporation, column (Merck, Chromolith Performance RP-18e (4.6×100 mm)), A solution: 5% acetonitrile, 0.1 M triethylamine acetate aqueous solution (TEAA), pH 7.0, B solution: acetonitrile, B%: 10%→50% (8 min., linear gradient); 60°C; 2 ml/min.; 254 nm) to collect the peak of the desired compound having 3,4-DBB groups.

When analyzed using reverse phase HPLC (LC-10VP from Shimadzu Corporation, column (Merck, Chromolith Performance RP-18e (4.6×100 mm)), A solution: 5% acetonitrile, 0.1 M triethylamine acetate aqueous solution (TEAA), pH 7.0, B solution: acetonitrile, B%: 10%→50% (8 min., linear gradient); 60°C; 2 ml/min.; 254 nm), the finally purified compound was eluted at 7.30 minutes (42.9 A₂₆₀ units) (λₘₐₓ (H₂O) = 258 nm). In addition, the compound was identified by negative ion ESI mass spectrometry (calculated value: 5538.75, measured value: 5538.30).

The base sequence of the compound is a sequence complementary to the nucleotide Nos. 137-148 region of human telomerase RNA (GenBank accession No. U86046).

### Example 12

### Synthesis of 3,4-DBB-T^{s}-T^{e2s}-A^{e2s}-G^{e2s}-G^{e2s}-G^{e2s}-T^{e2s}-T^{e2s}-A^{e2s}-G^{e2s}-A^{e2s}-C^{e2s}-A^{e2s}-A^{e2s}-CH₂CH₂OH (Exemplified Compound No. 150)

The compound of Example 12 having the desired sequence was synthesized in the same manner as the compound of Example 1 using 2.7 µmol of CPG identical to that in Example 1. Described in Example 12b of Japanese Patent Laid-Open No. 07-87982, 5'-O-[(3,4-dibenzyloxy)benzyl]-thymidine-3'-O-(2-cyanoethyl N,N-diisopropyl)phosphoramidite was lastly coupled. After deprotection, the purification was carried out using reverse phase HPLC (LC-10VP from Shimadzu Corporation, column (Merck, Chromolith Performance RP-18e (4.6×100 mm)), A solution: 5% acetonitrile, 0.1 M triethylamine acetate aqueous solution (TEAA), pH 7.0, B solution: acetonitrile, B%: 10%→40% (8 min., linear gradient); 60°C; 2 ml/min.; 254 nm) to collect the peak of the desired compound having 3,4-DBB groups.

When analyzed using ion exchange HPLC (LC-10VP from Shimadzu Corporation, column (Tosoh Corporation, DEAE-5PW (10×50 mm)), A solution: 20% acetonitrile aqueous solution, B solution: 20% acetonitrile, 67 mM phosphate buffer, 1.5 M potassium bromide aqueous solution, pH 6.8 B%: 10%→60% (8 min., linear gradient); 60°C; 2 ml/min.; 254 nm), the finally purified compound was eluted at 8.23 minutes (33.2 A₂₆₀ units) (λₘₐₓ (H₂O) = 259 nm). In addition, the compound was identified by negative ion ESI mass spectrometry (calculated value: 5176.45, measured value: 5175.63).

The base sequence of the compound is a sequence complementary to the nucleotide Nos. 137-149 region of human telomerase RNA (GenBank accession No. U86046).

### Example 13

### Synthesis of HOP(S)(OH)-O-T^{e2s}-A^{e2s}-G^{e2s}-G^{e2s}-G^{e2s}-T^{e2s}-T^{e2s}-A^{e2s}-G^{e2s}-A^{e2s}-C^{e2s}-A^{e2s}-A^{e2s}-CH₂CH₂OH (Exemplified Compound No. 159)

The compound of Example 13 having the desired sequence was synthesized in the same manner as the compound of Example 1 using 1.0 µmol of CPG identical to that in Example 1. However, phosphalink (from Applied Biosystems) was used according to the appended protocol in order to introduce a thiophosphate group into the 5' end. After deprotection, the purification was carried out using reverse phase HPLC (LC-10VP from Shimadzu Corporation, column (Merck, Chromolith Performance RP-18e (4.6×100 mm)), A solution: 5% acetonitrile, 0.1 M triethylamine acetate aqueous solution (TEAA), pH 7.0, B solution: acetonitrile, B%: 0%→80% (10 min., linear gradient); 60°C; 2 ml/min.; 254 nm) to collect the peak of the desired compound.

When analyzed using ion exchange HPLC (LC-10VP from Shimadzu Corporation, column (Tosoh Corporation, DEAE-5PW (10×50 mm)), A solution: 20% acetonitrile aqueous solution, B solution: 20% acetonitrile, 67 mM phosphate buffer, 1.5 M potassium bromide aqueous solution, pH 6.8 B%: 10%→80% (8 min., linear gradient); 60°C; 2 ml/min.; 254 nm), the finally purified compound was eluted at 7.27 minutes (33.6 A₂₆₀ units) (λₘₐₓ (H₂O) = 259 nm). In addition, the compound was identified by negative ion ESI mass spectrometry (calculated value: 5012.26, measured value: 5011.54).

The base sequence of the compound is a sequence complementary to the nucleotide Nos. 136-148 region of human telomerase RNA (GenBank accession No. U86046).

### Example 14

### Synthesis of C₁₇H₃₅C(O)O(CH₂)₂SP(O)(OH)-O-T^{e2s}-A^{e2s}-G^{e2s}-G^{e2s}-G^{e2s}-T^{e2s}-T^{e2s}-A^{e2s}-G^{e2s}-A^{e2s}-C^{e2s}-A^{e2s}-A^{e2s}-CH₂CH₂OH (Exemplified Compound No. 161)

The compound of Example 13 (18 A₂₆₀ units) was dissolved in 0.8 mL of DMF, to which diisopropylethylamine (4.96 µL) was then added, followed by adding thereto a solution consisting of 19 mg of 2-(stearoyloxy)ethyl bromide (Ackrman, et al., J. Am. Chem. Soc., 78: 6025 (1956)) dissolved, under heating, in 0.8 mL of DMF. The resultant solution was subjected to reaction at 42°C for 4 days. After the end of the reaction, it was washed thrice with 5 mL of n-hexane, and 2 mL of water was then added before removing the precipitated insoluble matter by filtration using a membrane filter, followed by purification employing reverse phase HPLC (LC-10VP from Shimadzu Corporation, column (Merck, Chromolith Performance RP-18e (4.6×100 mm)), A solution: 5% acetonitrile, 0.1 M triethylamine acetate aqueous solution (TEAA), pH 7.0, B solution: acetonitrile, B%: 0%→30% (10 min., linear gradient), 30%→80% (5 min), 60°C; 2 ml/min.; 254 nm) and then collecting the peak of the desired compound.

When analyzed using ion exchange HPLC (LC-10VP from Shimadzu Corporation, column (Tosoh Corporation, DEAE-5PW (10×50 mm)), A solution: 20% acetonitrile aqueous solution, B solution: 20% acetonitrile, 67 mM phosphate buffer, 1.5 M potassium bromide aqueous solution, pH 6.8 B%: 10%→80% (10 min., linear gradient); 60°C; 2 ml/min.; 254 nm), the finally purified compound was eluted at 8.23 minutes (3.9 A₂₆₀ units). In addition, the compound was identified by negative ion ESI mass spectrometry (calculated value: 5322.33, measured value: 5322.80).

The base sequence of the compound is a sequence complementary to the nucleotide Nos. 136-148 region of human telomerase RNA (GenBank accession No. U86046).

### Reference Example 1

### Synthesis of compound A

The synthesis was carried out at a scale of 1 µmol using an automated nucleic acid synthesizer (ABI model 394 DNA/RNA Synthesizer from Perkin Elmer). The concentrations of the solvent, reagent, and phosphoramidite in each synthesis cycle were the same as those for the synthesis of natural oligonucleotides. A 3% dichloroacetic acid/dichloromethane solvent (80 seconds) was used for detritylation; a 4,5-dicyanoimidazole/acetonitrile solvent (from Chem Genes, 900 seconds) for coupling; 0.3% xanthan hydride/pyridine:acetonitrile = 1:9 (v/v) (900 seconds) for sulfuration; and other solvents and reagents were obtained from Applied Biosystems. Non-natural phosphoroamidites as A^{a} and G^{a} moieties, used 6-N-benzoyl-3'-(trityl)amino-2',3'-dideoxyadenosine 5'-(2-cyanoethyl N,N-diisopropylphosphoramidite) and 2-N-isobutylyl-3'-(trityl)amino-2',3'-dideoxyguanosine 5'-(2-cyanoethyl N,N-diisopropylphosphoramidite) purchased from Transgenomic, respectively. As C^{a} and T^{a} moieties, 4-N-benzoyl-3'-(trityl)amino-2',3'-dideoxy-5-methylcytidine 5'-(2-cyanoethyl N,N-diisopropylphosphoramidite) and 3'-(trityl)amino-3'-deoxythymidine 5'-(2-cyanoethyl N,N-diisopropylphosphoramidite) were synthesized according to a reference (Nelson, J. S., et al., J. Org. Chem. (1997) 62: 7278-7287). A controlled pore glass (CPG) to which 3'-(trityl)amino-3'-deoxythymidine was bound at the 5' position via succinic acid was synthesized according to a reference ( Nelson, J. S., et al., J. Org. Chem. (1997) 62: 7278-7287) and used to synthesize the title compound without the trityl group.

A protected oligonucleotide analogue having the desired sequence was treated with concentrated ammonia water to cleave the oligomer from the support and simultaneously to remove protective cyanoethyl groups on the phosphorus atoms and protective groups on the nucleic acid bases. The solvent was distilled off under reduced pressure, followed by purifying the resultant residue using ion exchange HPLC (LC-10VP from Shimadzu Corporation, column (Tosoh Corporation, DEAE-5PW (10×50 mm)), A solution: 20% acetonitrile aqueous solution, B solution: 20% acetonitrile, 67 mM phosphate buffer, 1.5 M potassium bromide aqueous solution, pH 6.8 B%: 20%→40% (10 min., linear gradient); 60°C; 2 ml/min.; 254 nm) to collect the peak of a desired compound. The solvent was distilled off, followed by dissolving the residue in 2 ml of water to desalt by gel filtration using Sephadex G-25 (15×300 mm). A fraction corresponding to the desired compound was collected and distilled off, followed by dissolving the residue in 1 ml of water before filtration using a 0.45-µm filter (Millipore, Ultrafree-MC) to provide a desired oligonucleotide. When analyzed using ion exchange HPLC (LC-10VP from Shimadzu Corporation, column (Tosoh Corporation, DEAE-5PW (10×50 mm)), A solution: 20% acetonitrile aqueous solution, B solution: 20% acetonitrile, 67 mM phosphate buffer, 1.5 M potassium bromide aqueous solution, pH 6.8 B%: 20%→40% (10 min., linear gradient); 60°C; 2 ml/min.; 254 nm), this compound was eluted at 5.92 minutes. In addition, the compound was identified by negative ion ESI mass spectrometry (calculated value: 4216.71, measured value: 4215.81).

The compound has the same sequence as that of GRN163 described in Asai, et al., 2003, Cancer Research 63: 3931-3939, and uses a 5-methylcytosine base in place of a cytosine base.

The base sequence of the compound is a sequence complementary to the nucleotide Nos. 136-148 region of human telomerase RNA (GenBank accession No. U86046).

### Test Example 1: Test for determining melting temperature

Each of the compounds of the Examples was dissolved in a solution for determining melting temperature (Tm) (10 mM sodium phosphate buffer (pH 7.2)) so as to provide a final concentration of each of the compounds of 3.4 µM, which was further prepared so that an RNA oligonucleotide having the sequence of telomerase RNA (5'-UUGUCUAACCCUA-3') (3.4 µM) was present in the solution. The solution containing both of the strands (1.1 mL) was heated at 90°C for 5 minutes, followed by slow cooling to room temperature. The sample solution was determined using a spectrophotometer (Shimazu UV-3100PC). The sample was placed in a cell (cell thickness: 10 mm, cylindrical jacket type) and heated by circulated water heated in an incubator (Haake FE2 from EKO). The temperature was elevated from 20°C to 95°C while monitoring using a digital temperature indicator (SATO SK-1250MC), and absorbance was determined at 260 nm at 1°C intervals. The temperature at which the amount of change in absorbance per 1°C became maximal was denoted as Tm, and the compounds of the Examples were evaluated. The results are shown in Table 1.

**(Table 1) Melting temperature of each compound**

| Test compound | Tm (°C) |
|---|---|
| Compound of Example 1 | 85 |
| Compound of Example 2 | 87 |
| Compound of Example 3 | 74 |
| Compound of Example 4 | 62 |
| Compound of Example 5 | 52 |
| Compound of Example 6 | 71 |
| Compound of Reference Example 1 (Compound A) | 51 |

The compounds of these Examples had higher Tm values than that of compound A. This means that they more strongly bind to telomerase than compound A, which is known to have the activity of inhibiting telomerase, suggesting that the compounds of the Examples inhibit the enzyme activity of telomerase.

### Test Example 2: Stability test of oligonucleotides at pH 5.0

The compound of Example 1 (about 3.6 nmol) or compound A obtained in Reference Example 1 (about 3.6 nmol) was dissolved in 500 µL of a 20 mM sodium acetate aqueous solution (pH 5.0) and incubated at 37°C, and 73 µL thereof was sampled at fixed times, to which 27 µl of a sodium borate aqueous solution (pH 9.18) was then added for neutralization. These were analyzed using ion exchange HPLC (LC-10VP from Shimadzu Corporation, column (Tosoh Corporation, DEAE-5PW (10×50 mm)), A solution: 20% acetonitrile aqueous solution, B solution: 20% acetonitrile, 67 mM phosphate buffer, 1.5 M potassium bromide aqueous solution, pH 6.8 B%: 20%→70% (10 min., linear gradient); 60°C; 2 ml/min.; 254 nm), and the ratio between the area value (A) of each compound on the HPLC chart before the reaction and the area value thereof (B) after the reaction (B/A×100) was defined as the residual ratio (%) of each nucleotide. The results are shown in Figure 1.

As shown in the figure, the compound of Example 1 underwent no hydrolysis even after about 100 hours, whereas 60% of compound A was observed to be hydrolyzed after about 24 hours. These results demonstrated that the compound of Example 1 was extremely stable, compared to compound A, under acidic conditions similar to physiological conditions within cells into which they are incorporated by endocytosis.

### Formulation Example 1: Soft capsule

A mixture consisting of the compound of Example 1 placed in digestible oily matter, for example, soybean oil, cotton oil, or olive oil is prepared, and injected into gelatin using a positive replacement pump to provide a soft capsule containing 100 mg of active component, which is then washed before drying.

### Formulation Example 2: Tablet

According to a standard procedure, 100 mg of the compound of Example 2, 0.2 mg of colloidal silicon dioxide, 5 mg of magnesium stearate, 275 mg of microcrystalline cellulose, 11 mg of starch, and 98.8 mg of lactose are used for production.

In this respect, a coating is applied if desired.

### Formulation Example 3: Suspension

The suspension is produced so that 5 ml thereof contains 100 mg of the compound of Example 1, 100 mg of sodium carboxymethylcellulose, 5 mg of sodium benzoate, 1.0 g of sorbitol solution (Japanese Pharmacopeia), and 0.025 ml of vanillin.

### Formulation Example 4: Injection

In 10% by volume of propylene glycol was stirred 1.5% by weight of the compound of Example 2, to which water for injection was added to a fixed volume before sterilization for production.

### Formulation Example 5: Injection

N-(α-trimethylammonioacetyl)-didodecyl-D-glutamate chloride (final concentration: 30 nmol/ml), dilauroylphosphatidylcholine (final concentration: 60 nmol/ml), dioleoylphosphatidylethanolamine (final concentration: 60 nmol/ml), and 1.5% by weight of the compound of Example 2 were stirred in 10% by volume of propylene glycol, to which water for injection was added to a fixed volume before sterilization for production.

### Industrial Applicability

The compound of the present invention strongly binds to telomerase RNA, and is useful for treating or preventing diseases in which telomerase is involved, such as cancer.

## Claims

1. A compound having general formula (I) below:
E1-B1-B2-B3-B4-E2 (I)
(wherein E1 represents a group represented by the formula R1-, a group represented by the formula R1-B6-, or a group represented by the formula R1-B5-B6-;
E2 represents a group represented by the formula -B7-R2, a group represented by the formula -B8-B9-R2, a group represented by the formula -B8-B10-B11-R2, a group represented by the formula -B8-B10-B12-B13-R2, a group represented by the formula -B8-B10-B12-B14-B15-R2, a group represented by the formula -B8-B10-B12-B14-B16-B17-R2, or a group represented by the formula - B8-B10-B12-B14-B16-B18-B19-R2;
B4, B5, and B8 are identical or different, and each represents a group represented by formula: 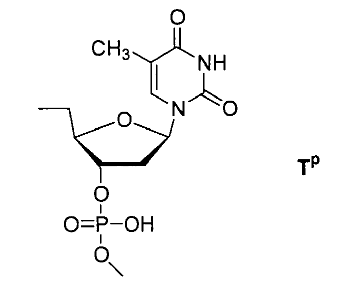 (hereinafter referred to as "T^{p}"), a group represented by formula: 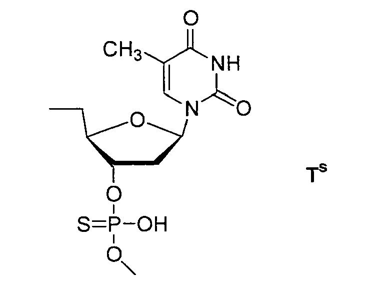 (hereinafter referred to as "T^{s}"), a group represented by general formula T^{ep}: 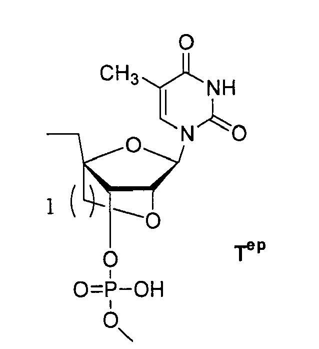 (where 1 represents an integer of 1 to 5), or a group represented by general formula T^{es}_{:} 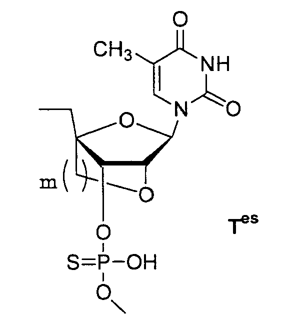 (where m represents an integer of 1 to 5);
B1, B2, B3, and B12 are identical or different, and each represents a group of formula: 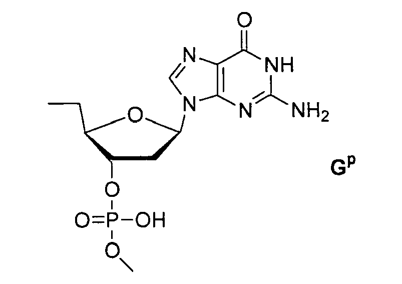 (hereinafter referred to as "G^{p}"), a group represented by formula: 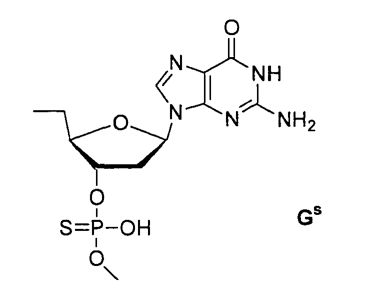 (hereinafter referred to as "G^{s}"), a group represented by general formula G^{ep}: 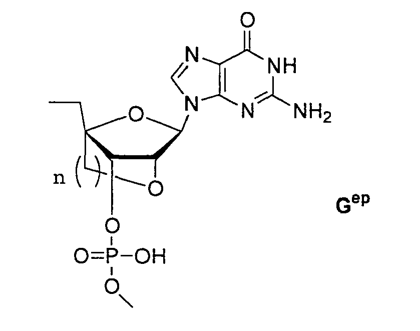 (where n represents an integer of 1 to 5), or a group represented by general formula G^{es}: 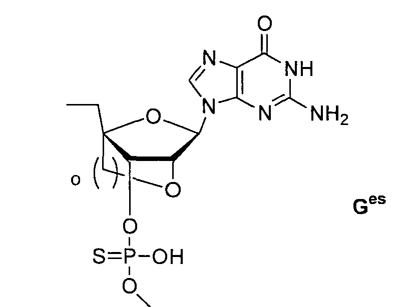 (where o represents an integer of 1 to 5);
B16 represents a group represented by formula: 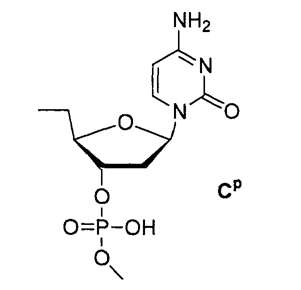 (hereinafter referred to as "C^{p}"), a group represented by formula: 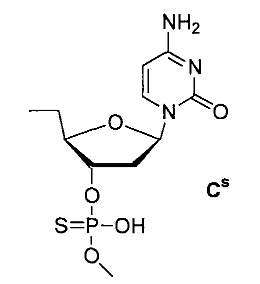 (hereinafter referred to as "C^{s}"), a group represented by general formula C^{ep}: 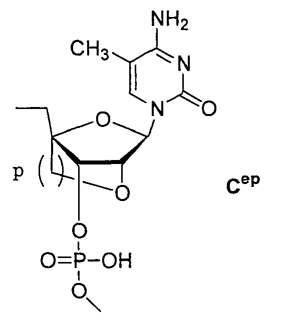 (where p represents an integer of 1 to 5), or a group represented by general formula C^{es}: 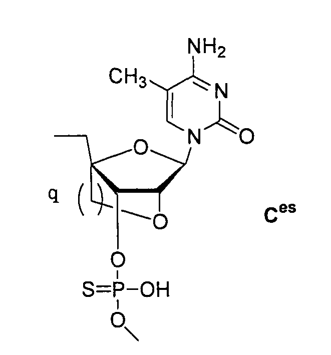 (where q represents an integer of 1 to 5);
B6, B10, B14, and B 18 are identical or different, and each represents a group represented by formula: 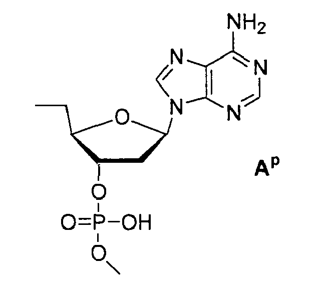 (hereinafter referred to as "A^{p}"), a group represented by formula: 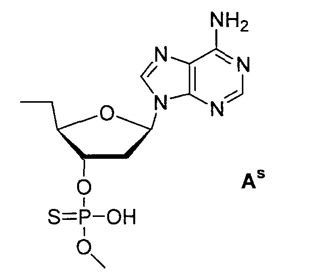 (hereinafter referred to as "A^{s}"), a group represented by general formula A^{ep}: 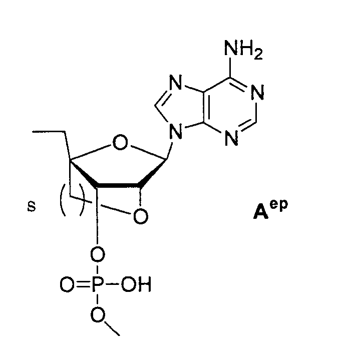 (where s represents an integer of 1 to 5), or a group represented by general formula A^{es}_{:} 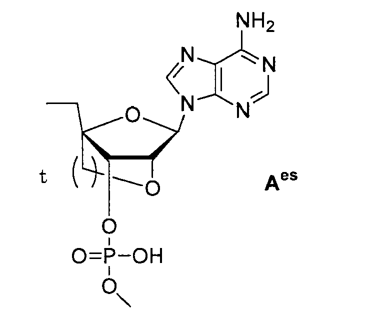 (where t represents an integer of 1 to 5);
B11 represents a group represented by formula: 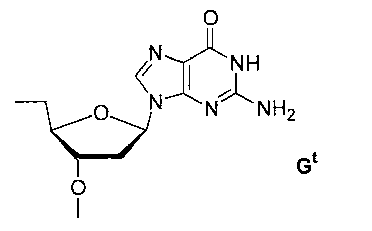 (hereinafter referred to as "G^{t}") or a group represented by general formula G^{et}: 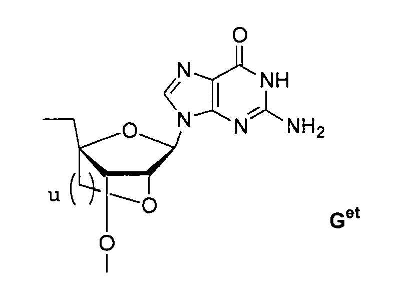 (where u represents an integer of 1 to 5);
B 15 represents a group represented by formula: 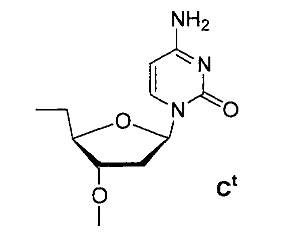 (hereinafter referred to as "C^{t}") or a group represented by general formula C^{et}: 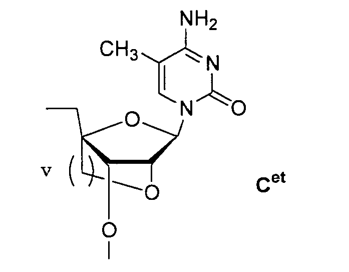 (where v represents an integer of 1 to 5);
B9, B 13, B 17, and B 19 are identical or different, and each represents a group represented by formula: 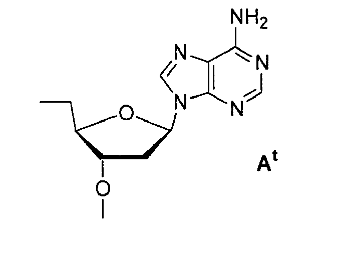 (hereinafter referred to as "A^{t}") or a group represented by general formula A^{et}: 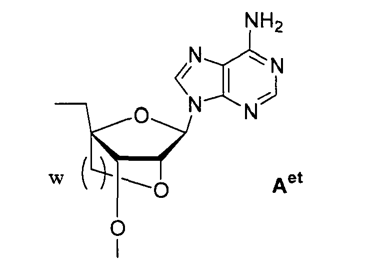 (where w represents an integer of 1 to 5);
B7 represents a group represented by formula: 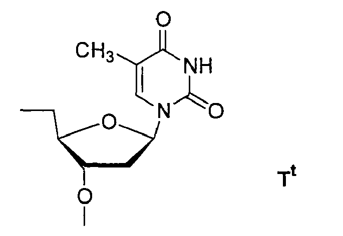 (hereinafter referred to as "T^{t}") or a group represented by general formula T^{et}: 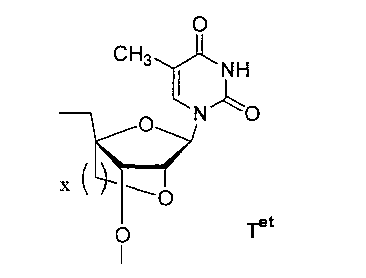 (where x represents an integer of 1 to 5);
R1 represents a hydroxyl group, a group represented by formula: 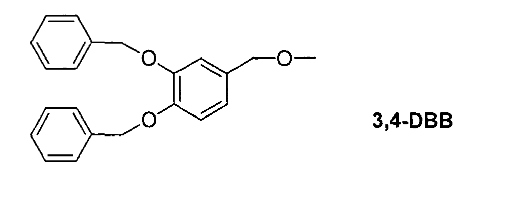 (hereinafter referred to as "3,4-DBB"), the group 3,4-DBB-(CH₂)₃-O-P(=O)(OH)-O-, the group 3,4-DBB-(CH₂)₃-O-P(=S)(OH)-O-, the group 3,4-DBB-(CH₂)₆-OP(=O)(OH)-O-, the group 3,4-DBB-(CH₂)₆-O-P(=S)(OH)-O-, the group C₁₅H₃₁C(O)O(CH₂)₂SP(=O)(OH)-O-, the group C₁₆H₃₃C(O)O(CH₂)₂SP(=O)(OH)-O-, the group C₁₇H₃₅C(O)O(CH₂)₂SP(=O)(OH)-O-, the group C₁₈H₃₇C(O)O(CH₂)₂SP(=O)(OH)-O-, or the group C₁₉H₃₉C(O)O(CH₂)₂SP(=O)(OH)-O-;
R2 represents a hydrogen atom, the group -P(=O)(OH)-O-CH₂-CH₂-OH, or the group -P(=S)(OH)-O-CH₂-CH₂-OH;
with the proviso that the following case is excluded:
B4, B5, and B8 are identical or different, and each is T^{p} or T^{s};
B1, B2, B3, and B12 are identical or different, and each is G^{p} or G^{s};
B16 is C^{p} or C^{s};
B6, B10, B 14, and B 18 are identical or different, and each is A^{p} or A^{s};
B11 is G^{t};
B15 is C^{t};
B9, B 13, B 17, and B 19 are each A^{t}; and
B7 is T^{t}),
or a pharmacologically acceptable salt thereof.

2. The compound according to claim 1, wherein B4, B5, and B8 are identical or different, and each is T^{ep} or T^{es};
B1, B2, B3, and B12 are identical or different, and each is G^{ep} or G^{es};
B16 is C^{ep} or C^{es};
B6, B10, B 14, and B 18 are identical or different, and each is A^{ep} or A^{es};
B11 is G^{et};
B 15 is C^{et};
B9, B13, B 17, and B 19 are each A^{et}; and
B7 is T^{et},
or a pharmacologically acceptable salt thereof.

3. The compound according to claim 1 or 2, wherein B4, B5, and B8 are each T^{es};
B1, B2 and B12 are each G^{es};
B16 is C^{es}; and
B6, B10, B14, and B 18 are each A^{es},
or a pharmacologically acceptable salt thereof.

4. The compound according to any one of claims 1 to 3, wherein E1 is a group represented by the formula R1-B5-B6-, or a pharmacologically acceptable salt thereof.

5. The compound according to any one of claims 1 to 3, wherein E1 is a group represented by the formula R1-B6-, or a pharmacologically acceptable salt thereof.

6. The compound according to any one of claims 1 to 3, wherein E1 is a group represented by the formula R1, or a pharmacologically acceptable salt thereof.

7. The compound according to any one of claims 1 to 6, wherein E2 is a group represented by the formula -B8-B10-B12-B14-B16-B18-B19-R2, or a pharmacologically acceptable salt thereof.

8. The compound according to any one of claims 1 to 6, wherein E2 is a group represented by the formula -B8-B10-B12-B14-B16-B17-R2, or a pharmacologically acceptable salt thereof.

9. The compound according to any one of claims 1 to 6, wherein E2 is a group represented by the formula -B8-B10-B12-B14-B15-R2, or a pharmacologically acceptable salt thereof.

10. The compound according to any one of claims 1 to 6, wherein E2 is a group represented by the formula -B8-B10-B12-B13-R2, or a pharmacologically acceptable salt thereof.

11. The compound according to any one of claims 1 to 6, wherein E2 is a group represented by the formula -B8-B10-B11-R2, or a pharmacologically acceptable salt thereof.

12. The compound according to any one of claims 1 to 6, wherein E2 is a group represented by the formula -B8-B9-R2, or a pharmacologically acceptable salt thereof.

13. The compound according to any one of claims 1 to 6, wherein E2 is a group represented by the formula -B7-R2, or a pharmacologically acceptable salt thereof.

14. The compound according to any one of claims 1 to 13, wherein R1 is a hydroxyl group, or a pharmacologically acceptable salt thereof.

15. The compound according to any one of claims 1 to 14, wherein R2 is the group -P(=O)(OH)-O-CH₂-CH₂-OH or the group -P(=S)(OH)-O-CH₂-CH₂-OH, or a pharmacologically acceptable salt thereof.

16. The compound according to any one of claims 1 to 14, wherein R2 is the group -P(=S)(OH)-O-CH₂-CH₂-OH, or a pharmacologically acceptable salt thereof.

17. The compound according to any one of claims 1 to 16, wherein 1, m, n, o, p, q, r , s, t, u, v, w, and x are identical or different and each is 1 or 2, or a pharmacologically acceptable salt thereof.

18. The compound according to any of claims 1 to 16, wherein 1, m, n, o, p, q, r , s, t, u, v, w, and x are identical, and each is 1 and 2, or a pharmacologically acceptable salt thereof.

19. The compound according to any of claims 1 to 16, wherein each of l, m, n, o, p, q, r , s, t, u, v, w, and x is 2, or a pharmacologically acceptable salt thereof.

20. A medicine containing a compound according to any one of claims 1 to 19 as an active ingredient.

21. An agent for preventing or treating diseases in which telomerase is involved, containing a compound according to any one of claims 1 to 19 as an active ingredient.

22. An agent for preventing or treating cancer, containing a compound according to any one of claims 1 to 19 as an active ingredient.

23. A telomerase inhibitor containing a compound according to any one of claims 1 to 19 as an active ingredient.

24. Use of a compound according to any one of claims 1 to 19 or a pharmacologically acceptable salt thereof for producing a medicinal composition.

25. A method for preventing or treating diseases in which telomerase is involved, which comprises administering, to a warm-blooded animal, a pharmacologically effective amount of a compound according to any one of claims 1 to 19 or a pharmacologically acceptable salt thereof.

26. A method for preventing or treating cancer, which comprises administering, to a warm-blooded animal, a pharmacologically effective amount of a compound according to any one of claims 1 to 19 or a pharmacologically acceptable salt thereof.

27. A method for inhibiting telomerase, which comprises administering, to a warm-blooded animal, a pharmacologically effective amount of a compound according to any one of claims 1 to 19 or a pharmacologically acceptable salt thereof.
